# FASCICULE DE BREVET EUROPEEN

(11) **EP 0 488 900 B1**
(45) Date de publication et mention de la délivrance du brevet: **28.02.2001**
(21) Numéro de dépôt: 91403243.8
(22) Date de dépôt: 29.11.1991
(51) Int. Cl.: C07K 14/00, C12P 21/02, C12N 5/10, C12N 1/19, C12N 15/19, A61K 38/00

(54) **Protéine présentant une activité de type cytokine, ADN recombinant, vecteur d'expression et hôtes permettant son obtention**
Protein mit Cytokinaktivität, Rekombinant-DNS, Expressionsvektor und Wirtszellen zu seiner Herstellung
Protein with cytokine activity, recombinant DNA, expression vector and hosts for obtaining it

(30) Priorité: 29.11.1990 FR 9014961
(43) Date de publication de la demande: 03.06.1992
(73) Titulaire: SANOFI-SYNTHELABO, 75013 Paris (FR)
(72) Inventeur: Caput, Daniel, F-31000 Toulouse (FR); Ferrara, Pascual, F-31290 Villefrance de Lauragais (FR); Miloux, Brigitte, F-31450 Montgiscard (FR); Minty, Adrian, F-31320 Mervilla (FR); Vita, Natalio, F-31650 Saint Orens (FR)
(74) Mandataire: Gillard, Marie-Louise

(56) Documents cités:
- WO-A-90/07863
- WO-A-90/08777

## Description

La présente invention a pour objet une nouvelle protéine présentant une activité de type cytokine, les outils de génie génétique pour la produire, à savoir un ADN recombinant, un vecteur d'expression portant cet ADN recombinant, les microorganismes procaryotes et les cellules eucaryotes contenant cet ADN recombinant ainsi qu'un médicament, utile notamment comme anticancéreux ou immunomodulateur, contenant à titre de principe actif cette protéine.

Il est bien connu que le système immunitaire comprend des éléments cellulaires et des substances solubles sécrétées par ces derniers, appelées cytokines. Celles-ci sont des protéines qui assurent la communication entre une cellule émettrice et une cellule cible appartenant, soit au système immunitaire, soit à un autre système biologique de l'organisme. Les cytokines ont en général une activité biologique dite pléiotropique : elles peuvent avoir de multiples actions sur la cellule cible : prolifération, différenciation, cytolyse, activation, chimiotactisme, etc... Plusieurs de ces molécules ont déjà trouvé des applications en thérapeutique : par exemple, l'interleukine-2 ou l'interféron-α utilisées pour le traitement de certaines tumeurs par immunothérapie et les facteurs myélopoiëtiques tels que le GCSF (Granulocyte Colony Stimulating Factor) ou le GMCSF (Granulocyte Monocyte Colony Stimulating Factor) qui stimulent la croissance et la différenciation des cellules sanguines et permettent ainsi l'enrichissement en ces dernières du sang appauvri en celles-ci suite à une chimiothérapie.

Une des premières cytokines découvertes est l'interleukine-1, à laquelle on a d'abord attribué une activité centrale dans l'inflammation : le chimiotactisme des neutrophiles, suite à des expériences montrant une activité de ce type in vivo après injection (J. Oppenheim et al, 1986, Imm. Today, 7, 45-56). On sait aujourd'hui que l'interleukine-1 stimule in vivo l'expression d'une autre cytokine chimiotactique vis-à-vis de neutrophiles, l'interleukine-8 (initialement appelée Neutrophil Chemotactic Factor NCF). Cette cytokine, dont la séquence d'acides aminés a été déterminée en 1987 après isolement et purification ainsi que par clonage et séquençage de son ADN complémentaire (K. Matsushima et al. 1988. J. Exp. Med., 1883-1893), est homologue à d'autres cytokines déjà connues à la date de sa découverte : les cytokines produites par les granules α des plaquettes telles que le PF4 (Platelet Factor 4) et le PBP (Platelet Basic Protein). La famille des protéines connues homologues à l'interleukine-8, appelée habituellement famille SIS (pour Small Induced Secreted proteins), s'est considérablement agrandie depuis 1987 [J. Oppenheim et al., 1991, Ann. Rev. Immun., 9, 617]. Elle compte aujourd'hui notamment: gro (également appelée MGSA : Melanoma Growth Stimulatory Activity) décrite par A. Anisowicz et al, 1987, Proc. Ntl. Acad. Sci. USA, 84, 7188-7192 et A. Richmond et al, 1988, EMBO J., 7, 2025-2033, RANTES (Regulated upon Activation Normal T Expressed and presumably Secreted) décrite par T. Schall et al, 1988, J. Imm., 141, 1018-1025, MIP-1 (Macrophage Inflammatory Protein 1) décrite par S.D. Wolpe et al, 1988, J. Exp. Med., 167, 570-581, MIP2 (Macrophage Inflammatory Protein 2) décrite par S.D. Wolpe et al, 1989, Proc. Ntl. Acad. Sci. USA, 86, 612-616 et MCP-1 (Monocyte Chemoattractant Protein 1 également appelée MCAF : Monocyte chemotactic and Activating Factor) décrite par Yoshimura et al, 1989, Proc. Ntl. Acad. Sci. USA, 84, 9233-9237 et K. Matsushima et al, 1989, J. Expr. Med., 169, 1485-1489.

La cytokine MCP-1, isolée d'une lignée de gliome par T. Yoshimura, référence ci-dessus, ainsi que d'une lignée monocytaire par K. Matsushima et al, référence ci-dessus, existe sous deux formes de masses moléculaires apparentes 13 et 15 kDa, appelées MCP-1α et MCP-1β qui semblent correspondre à des modifications post-traductionnelles [Y. Jiang et al., 1990, J. Biol. Chem., 265, 1318-321]. La cytokine MCP-1 a une activité chimiotactique pour les monocytes et les basophiles mais pas pour les neutrophiles (E.J. Leonard, 1990, Immunology Today, 11, 3, 97-101) et un effet stimulateur de l'activité cytostatique des monocytes sur certaines lignées tumorales (K. Matsushima et al, 1989, J. Exp. Med., 169, 1485-1490).

Les études de structures tridimensionnelles par spectroscopie de résonnance magnétique nucléaire ou diffraction des rayons X de l'interleukine-8 et de PF4 ont montré que ces deux cytokines ont la même conformation, avec un peptide carboxyterminal de 12 à 15 acides aminés en forme d'hélice a (R. St. Charles et al, 1988, J. Biol. Chem., 264, 4, 2092-2099 et G.M. Clore, 1990, Biochem., 29, 1689-1696). Selon G.M. Clore, la plupart des cytokines de la famille SIS ont une telle partie carboxy terminale en forme d'hélice a, le rôle de cette hélice restant à déterminer [C. Herbert et al., 1991, J. Biol. Chem., 266, 18989-994].

On a montré récemment que le peptide carboxy-terminal de 13 acides aminés peut présenter l'activité antiangiogénique (inhibition de la prolifération des vaisseaux sanguins) de la cytokine PF4 (T. Maione et al., 1990, Science, 247, 77-79). Selon D.G. Osterman, 1982, Biochem. Biophys. Res. Commun., 107, 130-135, ce peptide a une activité chimiotactique vis-à-vis des monocytes trente fois supérieure à celle de PF4.

La présente invention concerne une nouvelle protéine présentant une activité de type cytokine, caractérisée en ce qu'elle comprend la séquence (a1) suivante : 
Met Lys His Leu Asp Lys Lys Thr Gln Thr Pro Lys Leu
1               5                   10
 et immédiatement en amont de la séquence (a1), une partie de la séquence (a2) suivante :

Gln Pro Val Gly Ile Asn Thr Ser Thr Thr Cys Cys Tyr Arg Phe Ile
1               5                   10                  15
Asn Lys Lys Ile Pro Lys Gln Arg Leu Glu Ser Tyr Arg Arg Thr Thr
            20                  25                  30
Ser Ser His Cys Pro Arg Glu Ala Val Ile Phe Lys Thr Lys Leu Asp
        35                  40                  45
Lys Glu Ile Cys Ala Asp Pro Thr Gln Lys Trp Val Gln Asp Phe
    50                  55                  60

La partie de la séquence (a2) immédiatement en amont de la séquence (a1), peut être choisie parmi les séquences suivantes :

Gln Lys Trp Val Gln Asp Phe et Gln Asp Phe
1 5

De préférence la partie de la séquence (a2) immédiatement en amont de la séquence (al), est choisie parmi la séquence (a2) et les séquences (a3) et (a4) ci-après : 
(a3) Val Gly Ile Asn Thr Ser Thr Thr Cys Cys Tyr Arg Phe Ile Asn Lys
1 5 10 15
Lys Ile Pro Lys Gln Arg Leu Glu Ser Tyr Arg Arg Thr Thr Ser Ser
20 25 30
His Cys Pro Arg Glu Ala Val Ile Phe Lys Thr Lys Leu Asp Lys Glu
35 40 45
Ile Cys Ala Asp Pro Thr Gln Lys Trp Val Gln Asp Phe
50 55 60
 et

(a4) Lys Ile Pro Lys Gln Arg Leu Glu Ser Tyr Arg Arg Thr Thr Ser Ser
1 5 10 15
His Cys Pro Arg Glu Ala Val Ile Phe Lys Thr Lys Leu Asp Lys Glu
20 25 30
Ile Cys Ala Asp Pro Thr Gln Lys Trp Val Gln Asp Phe
35 40 45

La séquence (a2) est particulièrement appréciée, la protéine comportant alors éventuellement un blocage aminoterminal. La séquence de la protéine et le blocage aminoterminal correspondent probablement à ceux de la protéine exporté de cellules mononucléaires du sang périphérique dans des conditions stimulant l'expression de cytokines. Cette protéine présente, en ce qui concerne sa séquence d'acides aminés, une certaine ressemblance avec celle de la cytokine MCP-1, et, comme cette dernière elle possède une activité de type cytokine. Elle constitue un nouveau membre de la famille SIS.

Cette protéine est de préférence sous une forme qui présente une masse moléculaire apparente, déterminée par électrophorèse sur gel de polyacrylamide en présence de SDS de 9 ± 2, 11 ± 2, ou 16 ± 2 kDa. Cette protéine est avantageusement N-glycosylée en particulier lorsqu'elle est sous une forme de masse moléculaire apparente 16 ± 2 kDa. Cette protéine est avantageusement O-glycosylée en particulier lorsqu'elle est sous une forme de masse moléculaire apparente de 11 ± 2 kDa.

Cette protéine a de préférence un degré de pureté, déterminé par électrophorèse sur gel de polyacrylamide en présence de SDS et révélation au nitrate d'argent, supérieur à 90 %, et en particulier supérieur à 95 %.

L'invention a aussi pour objet un ADN recombinant caractérisé en ce qu'il code pour la protéine précédente, laquelle peut être alors obtenue à partir du lysat cellulaire ou, avantageusement, pour un précurseur de la protéine précédente. Ce précurseur comprend de préférence une séquence signal

Cette séquence signal, choisie en fonction de la cellule hôte, a pour fonction de permettre l'exportation de la protéine recombinante en dehors du cytoplasme, ce qui permet à la protéine recombinante de prendre une conformation proche de celle de la protéine naturelle et facilite considérablement sa purification. Cette séquence signal peut être clivée, soit en une seule étape par une signal-peptidase que libère la protéine mature, soit en plusieurs étapes lorsque cette séquence signal comprend en plus de la séquence éliminée par la signal-peptidase, appelée peptide signal ou séquence pré, une séquence éliminée plus tardivement au cours de un ou plusieurs évènements protéolytiques, appelée séquence pro.

Pour une expression dans les microorganismes procaryotes, tels que par exemple Escherichia coli, cette séquence signal peut être, soit une séquence dérivée d'un précurseur naturel d'une protéine exportée par un microorganisme procaryote (par exemple le peptide signal OMPa (Grayeb et al, 1984, EMBO Journal, 3, 2437-2442) ou celui de la phosphatase alcaline (Michaelis et al, J. Bact. 1983, 154, 366-374), soit une séquence non endogène provenant d'un précurseur eucaryote (par exemple le peptide signal de l'un des précurseurs naturels de l'hormone de croissance humaine), soit un peptide signal synthétique (par exemple celui décrit dans la demande de brevet français n° 2 636 643, de séquence :

Met Ala Pro Ser Gly Lys Ser Thr Leu Leu Leu Leu Phe Leu Leu Leu
1               5                   10                  15
Cys Leu Pro Ser Trp Asn Ala Gly Ala).
            20                  25

Pour une expression dans les cellules eucaryotes telles que les ascomycètes, par exemple la levure Saccharomyces cerevisiae ou le champignon filamenteux Cryphonectria parasitica, cette séquence signal est de préférence une séquence dérivée d'un précurseur naturel d'une protéine sécrétée par ces cellules, par exemple pour la levure le précurseur de l'invertase (demande de brevet EP-0123289) ou le précueseur de la séquence prépro de la phéromone alpha, (demande de brevet DK 2484/84), ou pour Cryphonectria parasitica, celui de la séquence prépro de l'endothiapepsine, de séquence :

Met Ser Ser Pro Leu Lys Asn Ala Leu Val Thr Ala Met Leu Ala Gly
1               5                   10                  15
Gly Ala Leu Ser Ser Pro Thr Lys Gln His Val Gly Ile Pro Val Asn
            20                  25                  30
Ala Ser Pro Glu Val Gly Pro Gly Lys Tyr Ser Phe Lys Gln Val Arg
        35                  40                  45
Asn Pro Asn Tyr Lys Phe Asn Gly Pro Leu Ser Val Lys Lys Thr Tyr
    50                  55                  60
Leu Lys Tyr Gly Val Pro Ile Pro Ala Trp Leu Glu Asp Ala Val Gln
65                  70                  75                  80
Asn Ser Thr Ser Gly Leu Ala Glu Arg
                85

Pour une expression dans les cellules animales, on utilise comme séquence signal, soit une séquence signal d'une protéine de cellule animale connue pour être exportée -par exemple le peptide signal de l'un des précurseurs naturels de l'hormone de croissance humaine, déjà connu pour permettre la sécrétion de l'interleukine-2 (cf. la demande de brevet français n° 2 619 711)-, soit l'une des trois séquences signal explicitées ci-dessous. 
(b1) Met Lys Ala Ser Ala Ala Leu Leu Cys Leu Leu Leu Thr Ala Ala Ala
1 5 10 15
Phe Ser Pro Gln Gly Leu Ala
20
 
(b2) Met Pro Ser Pro Ser Asn Met Lys Ala Ser Ala Ala Leu Leu Cys Leu
1 5 10 15
Leu Leu Thr Ala Ala Ala Phe Ser Pro Gln Gly Leu Ala
20 25
 et 
(b3) Met Trp Lys Pro Met Pro Ser Pro Ser Asn Met Lys Ala Ser Ala Ala
1 5 10 15
Leu Leu Cys Leu Leu Leu Thr Ala Ala Ala Phe Ser Pro Gln Gly Leu
20 25 30
Ala
 avantageusement codées par les séquences (Nb1), (Nb2) et (Nb3) suivantes : 
(Nb1)　ATGAAAGCCT CTGCAGCACT TCTGTGTCTG CTGCTCACAG
CAGCTGCTTT CAGCCCCCAG GGGCTTGCT
 
(Nb2) ATGCCCTCAC CCTCCAACAT GAAAGCCTCT GCAGCACTTC
TGTGTCTGCT GCTCACAGCA GCTGCTTTCA GCCCCCAGGG
GCTTGCT
 et

(Nb3) ATGTGGAAGC CCATGCCCTC ACCCTCCAAC ATGAAAGCCT
CTGCAGCACT TCTGTGTCTG CTGCTCACAG CAGCTGCTTT
CAGCCCCCAG GGGCTTGCT

La séquence nucléotidique codant pour la protéine mature est par exemple la séquence (Na2) suivante :

CAGCCAGTTG GGATTAATAC TTCAACTACC TGCTGCTACA
GATTTATCAA TAAGAAAATC CCTAAGCAGA GGCTGGAGAG
CTACAGAAGG ACCACCAGTA GCCACTGTCC CCGGGAAGCT
GTAATCTTCA AGACCAAACT GGACAAGGAG ATCTGTGCTG
ACCCCACACA GAAGTGGGTC CAGGACTTTA TGAAGCACCT
GGACAAGAAA ACCCAAACTC CAAAGCTT

L'invention concerne également un vecteur d'expression qui porte, avec les moyens nécessaires à son expression, l'ADN recombinant défini précédemment.

Pour une expression dans les micro-organismes procaryotes, en particulier dans Escherichia coli, l'ADN recombinant doit être inséré dans un vecteur d'expression comportant notamment un promoteur efficace, suivi d'un site de fixation des ribosomes en amont du gène à exprimer, ainsi qu'une séquence d'arrêt de transcription efficace en aval du gène à exprimer. Ce vecteur doit également comporter une origine de réplication et un marqueur de sélection. Toutes ces séquences doivent être choisies en fonction de la cellule hôte.

Pour une expression dans les cellules eucaryotes, le vecteur d'expression selon l'invention porte l'ADN recombinant défini précédemment avec les moyens nécessaires à son expression, ainsi qu'éventuellement les moyens nécessaires à sa réplication dans les cellules eucaryotes et/ou à la sélection des cellules transformées. De préférence, ce vecteur porte un marqueur de sélection, choisi par exemple pour complémenter une mutation des cellules eucaryotes réceptrices, qui permet la sélection des cellules qui ont intégré l'ADN recombinant en un nombre de copies élevé soit dans leur génome, soit dans un vecteur multicopie.

Pour une expression dans les cellules eucaryotes telles que la levure, par exemple Saccharomyces cerevisiae, il convient d'insérer l'ADN recombinant entre, d'une part, des séquences reconnues comme promoteur efficace, d'autre part, un terminateur de transcription. L'ensemble promoteur-séquence codante-terminateur, appelé cassette d'expression, est cloné soit dans un vecteur plasmidique monocopie ou polycopie pour la levure, soit intégré en multicopie dans le génome de la levure.

L'invention concerne aussi la levure qui contient, avec les moyens nécessaires à son expression, l'ADN recombinant défini précédemment.

L'invention a également trait à un procédé de préparation de la protéine ci-dessus, caractérisé en ce qu'il comprend une étape de culture de cette levure, suivie de l'isolement et de la purification de la protéine recombinante.

Pour une expression dans les cellules eucaryotes telles que celles des champignons filamenteux, du groupe des ascomycètes, par exemple ceux des genres Asperpillus, Neurospora, Podospora, Trichoderma ou Cryphonectria, le vecteur d'expression selon l'invention porte l'ADN recombinant défini précédemment avec les moyens nécessaires à son expression, et éventuellement un marqueur de sélection et/ou des séquences télomériques. Il est en effet possible de sélectionner les transformants ayant intégré un ADN d'intérêt à l'aide d'un marqueur de sélection situé soit sur le même vecteur que l'ADN d'intérêt, soit sur un autre vecteur, ces deux vecteurs étant alors introduits par cotransformation. L'ADN recombinant de l'invention peut être soit intégré au génome des champignons filamenteux, soit conservé sous forme extrachromosomique grâce à des séquences permettant la réplication et la partition de cet ADN.

Pour une expression dans les cellules animales, notamment dans les cellules d'ovaires de hamster chinois CHO, l'ADN recombinant est de préférence inséré dans un plasmide (par exemple dérivé du pBR322) comportant soit une seule unité d'expression dans laquelle est inséré l'ADN recombinant de l'invention et éventuellement un marqueur de sélection, devant un promoteur efficace, soit deux unités d'expression. La première unité d'expression comporte l'ADN recombinant ci-dessus, précédé d'un promoteur efficace (par exemple le promoteur précoce de SV40). La séquence autour de l'ATG d'initiation est de préférence choisie en fonction de la séquence consensus décrite par KOZAK (M. KOZAK (1978) Cell., 15, 1109-1123). Une séquence intronique, par exemple de l'intron de l'a-globine de souris, peut être insérée en amont de l'ADN recombinant ainsi qu'une séquence comportant un site de polyadénylation, par exemple une séquence de polyadénylation du SV40, en aval du gène recombinant. La deuxième unité d'expression comporte un marqueur de sélection, par exemple une séquence d'ADN codant pour la dihydrofolate réductase (enzyme ci-après abrégée DHFR). Le plasmide est transfecté dans les cellules animales, par exemple les cellules CHO dhfr (incapables d'exprimer la DHFR). Une lignée est sélectionnée pour sa résistance au méthotréxate : elle a intégré dans son génome un nombre élevé de copies de l'ADN recombinant et exprime ce dernier à un niveau suffisant.

L'invention a également trait aux cellules animales contenant, avec les moyens nécessaires à son expression, cet ADN recombinant. Ce dernier peut, par exemple, avoir été introduit dans les cellules par transfection par le vecteur d'expression ci-dessus, par infection au moyen d'un virus ou d'un rétro-virus le portant, ou par microinjection. Des cellules animales préférées sont des cellules CHO, en particulier les cellules CHO dhfr⁻ à partir desquelles il est possible d'obtenir des lignées hautement productrices en la protéine de l'invention. Les cellules COS constituent également un hôte avantageux pour l'obtention de cette protéine.

L'invention concerne aussi un procédé de préparation de la protéine définie précédemment qui comprend une étape de culture des cellules animales ci-dessus, suivie de l'isolement et de la purification de la protéine recombinante.

L'invention se rapporte également à la protéine recombinante susceptible d'être obtenue par un procédé qui comprend une étape de culture de ces cellules animales, suivie de l'isolement et de la purification de la protéine recombinante.

La protéine de l'invention est une cytokine possédant une activité chimiotactique vis-à-vis des monocytes, cellules qui peuvent inhiber la croissance des tumeurs (B.J. Rollins et al, 1991, Molecular and Cellular Biology, 11, 6, 3125-3131) et éliminer certains parasites tels que Leishmania major (S. Stenger et al, 1991, Eur. J. Immunol., 21, 327-33).

L'invention a donc également pour objet le médicament, utile notamment en cancérologie et dans le traitement de certains états infectieux, au cours desquels les défenses immunitaires sont affaiblies, suite par exemple à la présence de certains parasites (par exemple la leishmaniose, la lèpre ou le shagas), qui contient comme principe actif la protéine ou le peptide définis précédemment dans un excipient pharmaceutiquement acceptable. Ceux-ci peuvent être utilisés seuls ou en association avec d'autres agents actifs : par exemple une ou plusieurs autres cytokines.

L'invention sera mieux comprise à l'aide de l'exposé ci-après, divisé en sections, qui comprend des résultats expérimentaux et une discussion de ceux-ci. Certaines de ces sections concernent des expériences effectuées dans le but de réaliser l'invention, d'autres des exemples de réalisation de l'invention donnés bien sûr à titre purement illustratif.

Une grande partie de l'ensemble des techniques ci-après, bien connues de l'homme de l'art, est exposée en détail dans l'ouvrage de Sambrook et al : "Molecular cloning : a Laboratory manual" publié en 1989 par les éditions Cold Spring Harbor Press à New-York (2ème édition).

La description ci-après sera mieux comprises à l'aide des figures 1a, 1b, 2 à 5, 6a, 6b et 6c.

La figure 1a, représente une carte d'assemblage du plasmide pSE1, plasmide de clonage dans E. coli et d'expression dans les cellules animales, les sites ayant disparu par ligation étant notés entre parenthèses. Les symboles utilisés dans cette figure seront précisés lors de la description de ce plasmide (section 2).

La figure 1b représente la séquence du fragment synthétique "site liant à HIndIII"-HindIII utilisé dans l'assemblage du plasmide pSE1.

La figure 2 représente la séquence nucléotidique de l'ADNc NC28 et en vis-à-vis la séquence d'acides aminés déduite, les trois Met susceptibles d'initier la traduction étant soulignés, le site de clivage probable du peptide signal étant indiqué par un trait vertical et le site potentiel de N-glycosylation étant souligné en pointillés.

La figure 3 et la figure 4 représentent respectivement l'alignement d'après l'homologie maximale selon la méthode de Needleman et Wunsch, 1970, J. Mol. Biol., 48, 443-453 de la séquence d'acides aminés déduite de l'ADNc NC28 (ligne supérieure) et de la séquence d'acides aminés déduite de l'ADNc de la cytokine MCP-1 (ligne inférieure) et l'alignement selon cette méthode de l'ADNc NC28 (ligne supérieure) et de l'ADNc de la cytokine MCP-1 (ligne inférieure).

La figure 5 représente la séquence du fragment B utilisé dans la construction du plasmide pEMR617, vecteur d'expression dans la levure.

Les figures 6a, 6b et 6c sont relatives aux expériences de mise en évidence de l'activité chimiotactique.

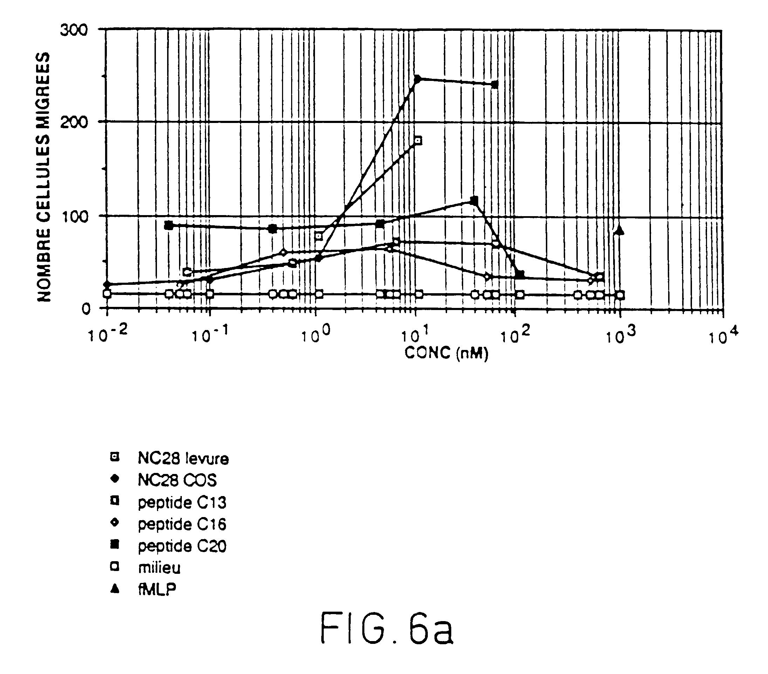

La figure 6a représente le nombre de cellules par champ microscopique en fonction de la concentration exprimée, en nM pour la protéine NC28 purifiée issue de la levure, la protéine NC28 purifiée issue de cellules COS, les peptides C13, C16, C20 et fMLP.

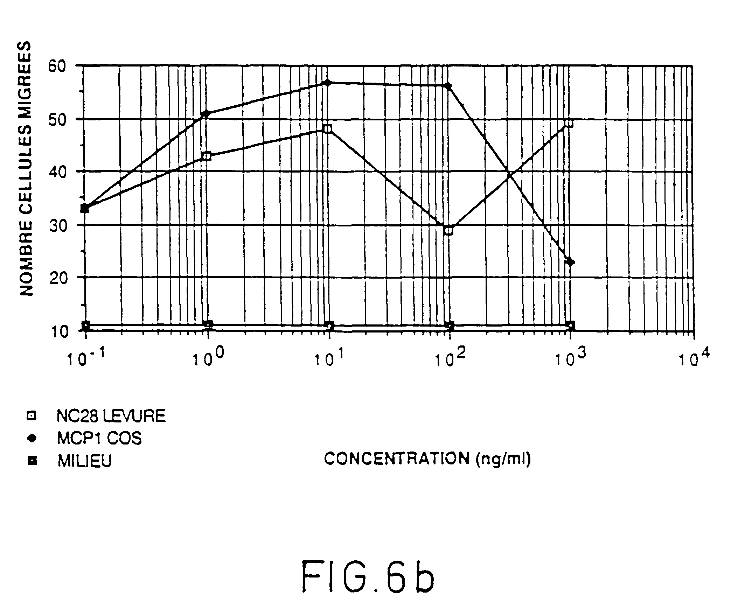

La figure 6b représente le nombre de cellules par champ microcospique en fonction de la concentration exprimée en ng/ml pour la protéine NC28 purifiée issue de levure et la cytokine MCP-1 purifiée issue des cellules COS.

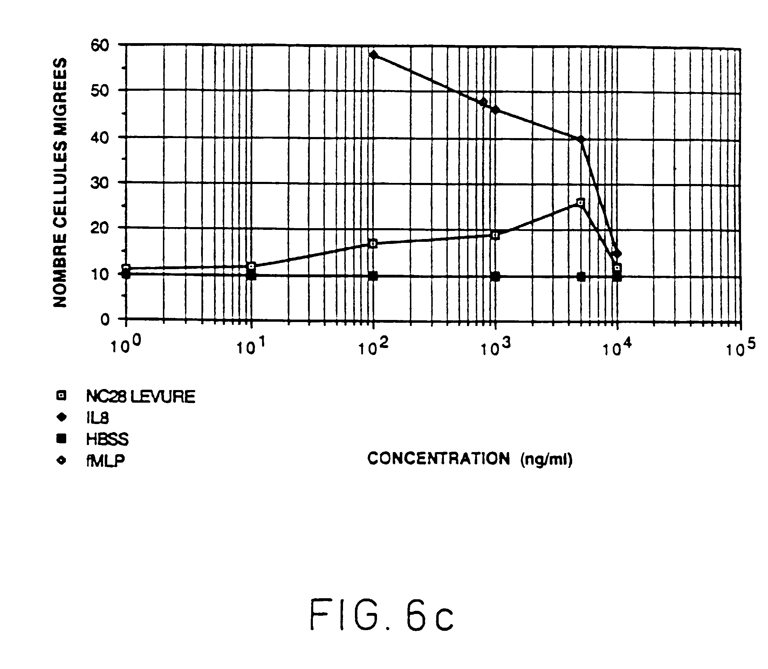

La figure 6c représente le nombre de cellules par champ microscopique en fonction de la concentration exprimée en ng/ml, pour la protéine NC28 purifiée issue de levure, la cytokine IL-8 et le peptide fMLP.

### SECTION 1 : Culture et stimulation à l'aide de PMA et PHA-P des cellules mononucléaires du sang périphérique. Préparation de l'ARN messager utilisé pour faire la banque d'ADN complémentaire

### 1) Culture et stimulation des cellules mononucléaires du sang périphérique :

A partir de poches de sang périphérique (prélevées sur trois volontaires sains dans un centre de transfusion sanguine), ayant préalablement subi une cytaphérèse et un gradient de Ficoll (Gauchat et al, 1989, Eur. J. Imm. 19, 1079) on prélève une fraction de cellules enrichie en cellules mononucléaires du sang périphérique PBMNC (peripheral blood mononuclear cells) de composition approximative suivante : 70 % de lymphocytes, 25 % de monocytes et 5 % de granulocytes (comptage des cellules à l'aide du compteur de cellules Coulter-Modèle S-Plus IV)

Les cellules sont recueillies dans un flacon de 250 ml, puis centrifugées pendant 10 min à 37°C. Le surnageant est éliminé et le culot de cellules est rincé avec 50 ml de milieu à base de glucose, sels minéraux, acides aminés et vitamines, appelé milieu RPMI (milieu RPMI 1640 de Gibco BRL), puis de nouveau centrifugé dans les mêmes conditions.

Le culot de cellules est alors repris avec 500 ml du milieu RPMI complété avec 10 % de sérum de veau foetal (Gibco BRL- réf. 013-06290H), additionné de 10 unités de pénicilline et de 10 µg de streptomycine (solution de pénicilline/streptomycine de Gibco réf. 043-05140D) par ml de milieu ainsi que de L-glutamine (Gibco BRL-réf. 043-05030D) jusqu'à 2mM final.

Une partie de la suspension cellulaire est répartie en vue de la séparation des cellules adhérentes et des cellules non adhérentes, à raison d'environ 100 ml par boîte, dans quatre grandes boîtes de culture carrées (245 x 245 x 20 mm-Nunc - réf. 166508) et incubée pendant 1 h à 37°C. On sait en effet que la plupart des cellules monocytaires adhérent à la boîte de culture tandis que la plupart des cellules lymphocytaires restent en suspension.

Les cellules non adhérentes sont aspirées à l'aide d'une pipette et cultivées dans des flacons de culture du type Falcon de surface 175 cm² en présence de milieu RPMI complété comme décrit ci-dessus, additionné de 10 ng/ml de phorbol myristate-2 acétate-3 (PMA) (Sigma-réf. P8139) et de 5µg/ml de phytohémaglutinine (PHA-P) (Sigma-réf. L8754), à 37°C en présence de 5 % de CO₂ pendant 24 h.

Sur les cellules adhérentes on rajoute 100 ml de milieu RPMI complété comme décrit ci-dessus additionné de 10 ng/ml de PMA et de 5 µg/ml de PHA-P. Les cellules sont incubées à 37°C en présence de 5 % (v/v) de CO₂ pendant 5 h.

Le reste de la suspension cellulaire, appelé ci-après les cellules totales, est réparti dans 4 grandes boîtes de culture carrées et incubé en présence de milieu RPMI complété comme décrit ci-dessus, additionné de 10 ng/ml de PMA et 5 µg/ml de PHA-P à 37°C en présence de 5 % (v/v) de CO₂ pendant 5 h pour les deux premières boîtes et 24 h pour les deux autres.

2 h environ avant la fin de l'incubation on ajoute dans le milieu de culture de ces différentes cellules 10µg/ml de cycloheximide (Sigma réf.C6255) (inhibiteur de traduction qui augmente la stabilité des ARN des cytokines : cf. Lindsten et al, 1989, Science 244, 339-344) et l'incubation est poursuivie pendant 2 h à 37°C.

### 2) Préparation de l'ARN messager :

### a) Extraction de l'ARN messager

Les cellules sont récupérées de la façon suivante :
- les cellules adhérentes sont lavées 2 fois avec du tampon PBS (Phosphate buffered saline réf. 04104040-Gibco BRL) puis grattées avec un grattoir en caoutchouc et centrifugées. On obtient ainsi un culot cellulaire, appelé culot A.
- pour les cellules non adhérentes, après agitation du flacon contenant la suspension des cellules, la suspension cellulaire est prélevée et centrifugée. On obtient ainsi un culot cellulaire, appelé culot cellulaire NA.
- pour les cellules totales, la fraction adhérente est lavée 2 fois avec du tampon PBS et grattée comme précédemment puis centrifugée. La fraction non adhérente est centrifugée. Les deux culots cellulaires obtenus seront réunis par la suite. Leur réunion est appelée culot cellulaire T(5 h) pour les cellules totales incubées pendant 5 h et T(24 h) pour les cellules totales incubées pendant 24 h.

Les culots cellulaires A, NA, T(5 h) et T(24 h) sont congelés et conservés à -80°C.

Chaque culot cellulaire congelé est mis en suspension dans le tampon de lyse de composition suivante : guanidine- thiocyanate 5M ; Tris-(hydroxyméthyl)-aminométhane 50mM pH 7,5 EDTA 10mM. La suspension est soniquée à l'aide d'un sonicateur Ultra Turax n° 231 256 (Janke et Kunkel) à puissance maximale pendant 4 cycles de 20 s. On ajoute du β-mercaptoéthanol jusqu'à 0,2M et on refait un cycle de sonication de 30 s. On ajoute du chlorure de lithium jusqu'à 3M. On refroidit la suspension à 4°C et on la laisse reposer à cette température pendant 48 h. L'ARN est ensuite isolé par centrifugation pendant 60 min. Le culot d'ARN est lavé une fois avec une solution de chlorure de lithium 3M, recentrifugé, puis repris dans un tampon de composition suivante : SDS 1 %, EDTA 5mM et Tris HCl 10mM pH 7,5, additionné de 1 mg/ml de protéinase K (Boehringer Mannheim, GmbH). Après incubation à 40°C pendant 1 h la solution d'ARN est extraite avec un mélange phénol/chloroforme. On précipite à -20°C l'ARN contenu dans la phase aqueuse à l'aide d'une solution d'acétate d'ammonium 0,3M final et 2,5 volumes d'éthanol. On centrifuge à 15 000 g pendant 30 min et on garde le culot.

### b) Purification de la fraction poly A⁺ de l'ARN

Le culot est repris dans 1 ml de tampon de composition Tris-HCl 10mM pH 7,5 EDTA 1mM, appelé tampon TE et mis en suspension par agitation au vortex. L'oligo dT-cellulose type 3 (commercialisé par Collaborative Research Inc, Biomedicals Product Division) est préparé suivant les recommandations du fabricant. L'ARN est déposé sur l'oligo dT-cellulose, agité doucement pour mettre en suspension les billes, puis chauffé pendant 1 min à 65°C.

La suspension est ajustée à 0,5 M NaCl, puis mise à agiter doucement pendant 10 min. La suspension est alors centrifugée pendant 1 min à 1000 g, le surnageant est éliminé, le culot est lavé 2 fois avec 1 ml de tampon TE contenant 0,5 M NaCl. Les surnageants sont éliminés. L'élution de la fraction polyadénylée des ARN (constituée des ARN messagers) est obtenue par suspension des billes dans 1 ml de tampon TE, puis chauffage de cette suspension à 60°C pendant 1 min, suivie d'une agitation pendant 10 min sur plaque basculante. On centrifuge ensuite pendant 1 min à 1000 g, ce qui permet de récupérer le surnageant contenant des ARN messagers libres en solution. L'ensemble des opérations ci-dessus (à partir de l'élution) est répété 2 fois. Les surnageants ainsi obtenus sont rassemblés, on élimine les billes résiduelles par centrifugation et on précipite le surnageant avec 3 volumes d'éthanol et une solution de NaCl à 0,3M final.

On obtient ainsi à partir des culots cellulaires A, NA, T(5 h) et T(24 h), quatre échantillons d'ARN-poly A⁺, nommés par la suite ARN-poly A⁺-A, ARN-poly A⁺-NA; ARN-polyA ⁺-T(5 h) et ARN-poly A⁺-T(24 h).

### SECTION 2 : Préparation d'une banque d'ADN complémentaire enrichie en séquences spécifiques des cellules mononucléaires du sang périphérique

### 1) Construction du vecteur de clonage pSE1 :

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par T. Maniatis, EF. Fritsch et J. Sambrook dans "Molecular Cloning, a Laboratory manual" (Cold Spring Harbor Laboratory, 1984). La synthèse des oligonucléotides est réalisée à l'aide d'un synthétiseur d'ADN Biosearch 8700.

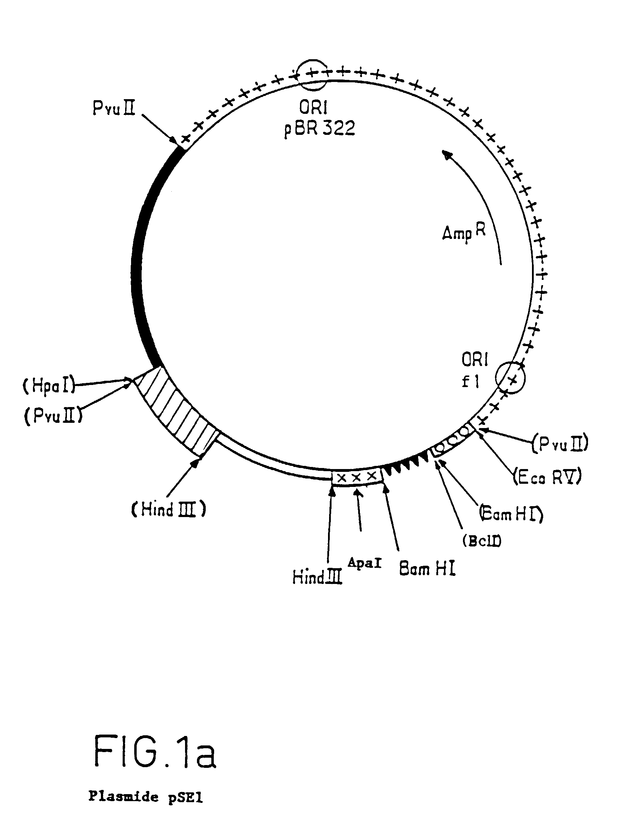

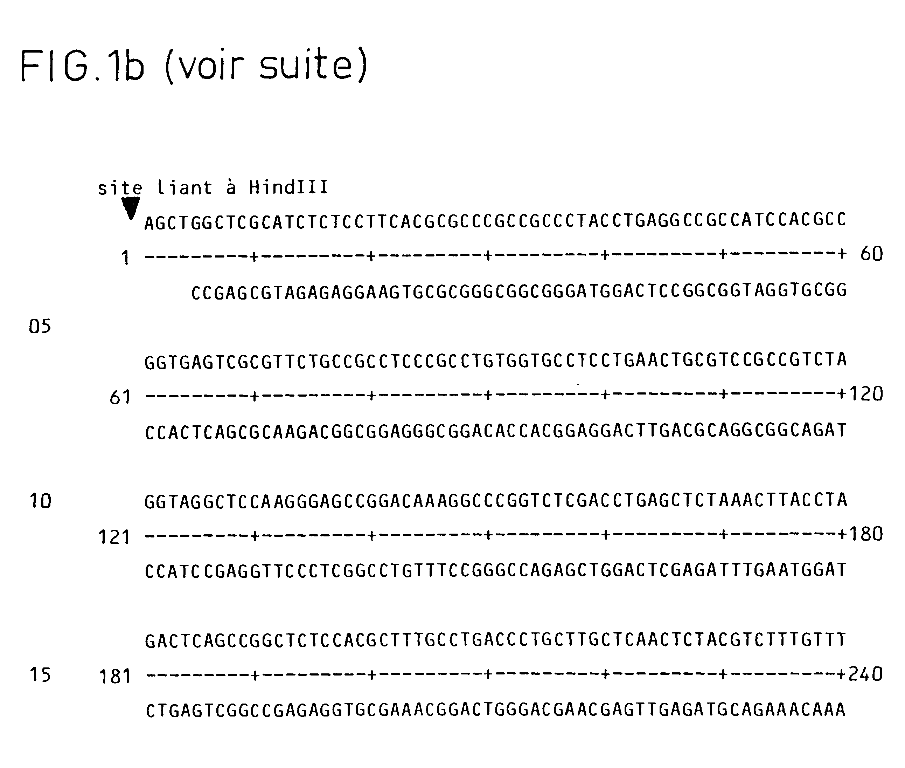

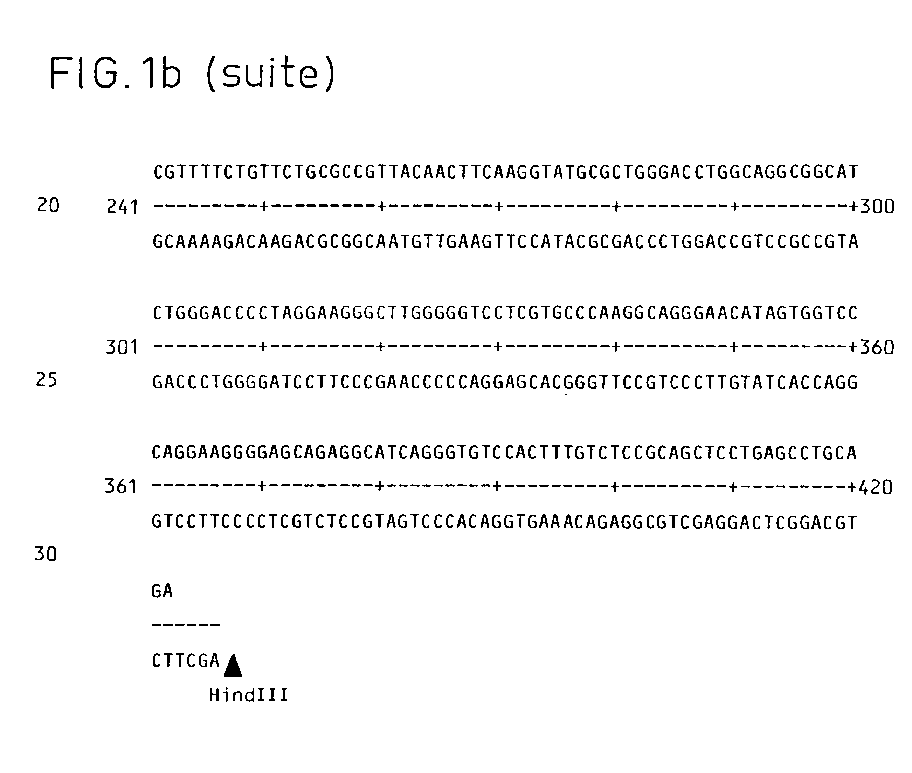

La description ci-après sera mieux comprise en référence à la figure 1ₐ.

Ce plasmide a été construit par ligations successives des éléments suivants :
a)- un fragment PvuII-PvuII - symbolisé par +++++++ sur la figure 1ₐ - de 2525pb, obtenu par digestion complète du plasmide pTZ18R (Pharmacia) à l'aide de l'enzyme de restriction PvuII. Ce fragment contient l'origine de réplication du phage f1 (notée ORI F1 sur la figure 1ₐ), un gène (noté Amp^{R} sur la figure 1ₐ) portant la résistance à l'ampicilline et l'origine de réplication (notée ORI pBR322 sur la figure 1ₐ) permettant la réplication de ce plasmide dans E. coli. Le premier site franc PvuII disparaît par ligation avec le site franc EcoRV (qui disparaît également) du fragment décrit en g).
b)- un fragment PvuII-HpaI - symbolisé par 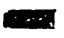 sur la figure 1ₐ - de 1060 pb de l'ADN d'adénovirus type 5 entre les positions 11299 (site de restriction PvuII) et 10239 (site de restriction HpaI) (Dekker et Van Ormondt, Gene 27, 1984, 115-120) contenant l'information pour les ARN VA-I et VA-II. Le site franc HpaI disparaît par ligation avec le site franc PvuII (qui disparaît également) du fragment décrit en c). Le site ApaI en position 11 218 a été enlevé par clivage à l'aide de l'enzyme ApaI, traitement à l'aide de l'exonucléase : ADN polymérase du phage T4 et religation.
c)- un fragment PvuII-HindIII - symbolisé par 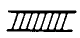 sur la figure 1ₐ - de 344 pb, issu de l'ADN du virus SV40 obtenu par digestion complète à l'aide des enzymes de restriction PvuII et HindIII. Ce fragment comporte l'origine de réplication et le promoteur précoce de l'ADN du virus SV40 (réf. B.J. Byrne et al. Proc. Ntl. Acad. Sci. USA (1983), 80, 721-725).
   Le site HindIII disparaît par ligation avec le site liant à HindIII du fragment décrit en d).
d)- un fragment synthétique "site liant à HindIII" - HindIII - symbolisé par 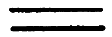 sur la figure 1ₐ - de 419 pb dont la séquence, donnée sur la figure 1_{b}, contient une séquence proche de la séquence 5' non traduite du virus HTLV1 (R. WEISS et al, "Molecular Biology of Tumor Viruses" - part 2-2^{e} ed - 1985 - Cold Spring Harbor Laboratory - p. 1057) et l'intron distal du gène de l'α-globine de souris (Y. Nishioka et al, 1979, Cell, 18, 875-882).
e)- un fragment synthétique HindIII-"site liant à BamHI" - symbolisé par XXXXXXX sur la figure 1ₐ - contenant le promoteur de l'ARN-polymérase du phage T7 ainsi qu'un polylinker contenant notamment les sites de clonage ApaI et BamHI 
ApaI
AGCTTGTCGACTAATACGACTCACTATAGGGCGGCCGCGGGCCCCT
ACAGCTGATTATGCTGAGTGATATCCCGCCGGCGCCCGGGGA
HindIII BamHI site liant à BamHI
GCAGGAATTCGGATCCCCCGGGTGACTGACT
CGTCCTTAAGCCTAGGGGGCCCACTGACTGACTAG
f)- un fragment BamHI-BclI de 240 pb - représenté par 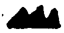 sur la figure 1ₐ -, petit fragment obtenu par digestion complète à l'aide des enzymes BclI et BamHI de l'ADN du virus SV40 qui contient le site de polyadénylation tardif de ce dernier. (M. Fitzgerald et al. Cell, 24, 1981, 251-260). Les sites BamHI et BclI disparaissent par ligations avec respectivement le "site liant à BamHI" du fragment décrit en e) et le site BamHI (qui disparaît également) du fragment décrit en g).
g)- un fragment BamHI-EcoRV - symbolisé par OOOOOO sur la figure 1ₐ- de 190 pb, petit fragment issu du plasmide pBR322 après digestion complète à l'aide des enzymes EcoRV et BamHI.

Le plasmide pSE1 comporte donc les éléments nécessaires pour son utilisation comme vecteur de clonage dans E. coli (origine de replication dans E. coli et gène de résistance à l'ampicilline, provenant du plasmide pTZ18R) ainsi que comme vecteur d'expression dans les cellules animales (promoteur, intron, site de polyadénylation, origine de replication du virus SV40), et pour sa copie en simple brin dans un but de séquençage (origine de réplication du phage f1 ).

### 2) Constitution d'une banque d'ADN complémentaire enrichie en séquences spécifiques des cellules mononucléaires du sang périphérique :

La technique de clonage utilisée est celle décrite par Caput et al, (technique de l'amorce-adaptateur (Primer-adapter) : Caput et al, Proc. Natl. Acad. Sci. (U.S.A.), 1986, 83, 1670-1674).

Elle consiste d'une part à digérer le vecteur pSE1 par ApaI, ajouter une queue de polydC sur l'extrémité 3' protubérante, puis à digérer les plasmides ainsi obtenus par l'endonucléase BamHI. Le fragment correspondant au vecteur est purifié sur colonne de Sépharose CL4B (Pharmacia). Il comprend donc une queue polydC à une extrémité, l'autre extrémité étant cohésive, du type BamHI.

D'autre part, les ARN polyA⁺ obtenus à l'issue de la section 1 sont soumis à la transcription inverse à partir d'une amorce dont la séquence est la suivante : 5' GATCCGGGCCCTTTTTTTTTTTTT 3' Ainsi, les ADNc présentent à leur extrémité 5' la séquence GATCC complémentaire de l'extrémité cohésive BamHI.

Les hybrides ARN-ADN obtenus par action de la transcriptase inverse sont soumis à une hydrolyse alcaline qui permet de se débarrasser de l'ARN. Les ADNc simple brin sont alors soumis à un traitement à la terminale transférase, de façon à ajouter des polydG en 3' et purifiés par 2 cycles sur colonne de sépharose CL4B.

Ces ADNc sont hybridés avec de l'ARN-polyA⁺ provenant de cellules de la lignée COS3 (lignée de cellules de reins de singe exprimant l'antigène T du virus SV40 : cf. Y. Gluzman, 1981, Cell, 23, 175-182 préparées comme décrit dans la Section 1.2).

Les ADNc non hybridés sont isolés (fraction enrichie en ADN complémentaire aux ARN messagers spécifiques des cellules mononucléaires du sang périphérique).

Ces ADNc sont insérés sous forme simple brin dans le vecteur pSE1. Un second oligonucléotide (l'adaptateur) complémentaire de l'amorce est nécessaire pour générer un site BamHI à l'extrémité 5' des ADNc. Après hybridation du vecteur, de l'ADNc et de l'adaptateur, les molécules recombinantes sont circularisées par l'action de la ligase du phage T4. Les régions simple brin sont alors réparées grâce à l'ADN polymérase du phage T4. Le pool de plasmides ainsi obtenu sert à transformer la souche E. coli MC 1061 (Casabadan et S. Cohen, J. Bact. (1980) 143, 971-980) par électroporation.

### Protocole de préparation de la banque d'ADN complémentaire

### a) Préparation de l'ADN complémentaire

A partir de 5 µg des ARN-poly A⁺ de cellules mononucléaires de sang périphérique obtenus à l'issue de la section 1 de composition suivante : ARN-poly A⁺ A : 0,5 µg, ARN-poly A⁺ NA: 2 µg, ARN-poly A⁺ T(5 h): 2 µg et ARN-poly A⁺ T(24 h) : 0,5 µg, on prépare l'ADN complémentaire simple-brin marqué au ³²P-dCTP (l'ADN complémentaire obtenu présente une activité spécifique de 3000 dpm/ng) avec l'amorce synthétique de séquence suivante (comprenant un site BamHI) : 5' GATCCGGGCCCTTTTTTTTTTTTTT 3' dans un volume de 100µl. Après 30 min d'incubation à 46°C avec 100 unités de l'enzyme transcriptase inverse (Genofit-E1 022) on ajoute 4µl d'EDTA 0,5M. On extrait une première fois avec du phénol (saturé en tampon TE) puis une seconde fois avec du chloroforme. On ajoute 10 µg d'ARN de transfert de foie de veau, 1/10ème de volume d'une solution d'acétate d'ammonium 10M et 2,5 volumes d'éthanol pour précipiter l'ADN complémentaire. On centrifuge, on dissout le culot dans 30µl de tampon TE puis on retire les petites molécules telles que sels, phénol et chloroforme par chromatographie d'exclusion sur une colonne de polyacrylamide P10 (Biogel P10-200-400mesh, réf. 1501050-Biorad).

### b) Hydrolyse alcaline de la matrice ARN

On ajoute 4,6µl d'une solution de NaOH 2N, on incube pendant 30 min à 68°C, puis on ajoute 4,6µl d'acide acétique 2N et on fait passer la solution obtenue sur une colonne de polyacrylamide P10.

### c) Addition homopolymérique de dG

On allonge l'ADN complémentaire en 3' avec une "queue" de dG avec 66 unités de l'enzyme terminale transférase (Pharmacia 27073001). On incube pendant 30 min à 37°C, puis on ajoute 4 µl d'EDTA 0,5M.

### d) Purification sur colonne de sépharose CL4B

Afin d'éliminer l'amorce synthétique, on purifie l'ADN complémentaire sur deux colonnes successives de 1 ml de sépharose CL4B (Pharmacia), équilibrées avec une solution NaOH 30mM/EDTA 2mM.

Les trois premières fractions radioactives (d'environ 80µl chacune) sont regroupées et précipitées avec 1/10ème de volume d'une solution d'acétate d'ammonium et 2,5 volumes d'éthanol. La quantité d'ADN complémentaire est de 1 µg.

### e) Hybridation

Le culot d'ADN complémentaire est mis en suspension dans 25 µl de tampon TE, on ajoute 15µg d'ARN-polyA⁺ extrait de cellules de la lignée COS, puis 1/10ème de volume d'une solution de NaCl 3M, 2,5 volumes d'éthanol et on laisse précipiter à -20°C.

On centrifuge, on lave à l'éthanol 70 %, on sèche, on dissout le culot dans 5 µl de tampon de composition suivante : Tris-HCl 0,1M pH 7,5 ; NaCl 0,3M, EDTA 1mM, on met la solution obtenue dans un tube capillaire que l'on scelle, puis on incube à 65°C pendant 40 h.

On dilue le contenu du capillaire dans 100µl de tampon TE auquel on ajoute 300µl de tampon phosphate de sodium 50mM pH6,8. On fait passer la solution obtenue sur une colonne d'hydroxyapatite (Biorad réf. 130.0520) à 60°C, équilibrée avec ce tampon phosphate. On sépare le simple brin (l'ADN complémentaire non-hybridé) et le double brin (ARN messager de COS hybridé à l'ADN complémentaire) par un gradient de tampon phosphate de 0,1M à 0,2M à travers la colonne d'hydroxyapatite. On regroupe les fractions correspondant à l'ADN complémentaire simple brin (25 % en poids de l'ADNc élué, ce qui correspond à un enrichissement d'environ 4 fois en séquences spécifiques de cellules mononucléaires du sang périphérique), on ajoute 20 µg d'ARN de transfert, on précipite le volume total avec 1/10ème de volume d'une solution d'acétate d'ammonium 10M et 2,5 volumes d'éthanol. On centrifuge, le culot est dissout dans 200 µl de TE, on retire le phosphate résiduel sur polyacrylamide P10, on précipite de nouveau avec 1/10^{ème} de volume d'une solution d'acétate d'ammonium 10M et 2,5 volumes d'éthanol.

Le culot est dissous dans 30 µl d'une solution de NaOH 30mM ; EDTA 2mM. On charge l'ADN complémentaire sur une colonne de sépharose CL4B (Pharmacia) de 1 ml, équilibrée avec une solution de NaOH 30mM ; EDTA 2mM, afin d'éliminer le reste d'amorce synthétique. On regroupe les 3 premières fractions radioactives d'environ 80 µl chacune. On précipite l'ADNc contenu dans ces fractions avec 1/10^{ème} de volume d'une solution d'acétate d'ammonium 10 M et 2,5 volume d'éthanol. La quantité d'ADN complémentaire ainsi récupérée est de 20 ng.

### f) Appariement du vecteur de clonage pSE1 et de l'ADN complémentaire en présence de l'adaptateur

On centrifuge, le culot est dissous dans 33 µl de tampon TE, on ajoute 5 µl (125 ng) de vecteur de clonage pSE1, 1 µl (120 ng) de l'adaptateur de séquence suivante (comprenant un site ApaI) : 5' AAAAAAAAAAAAAAGGGCCCG 3' 10µl d'une solution de NaCl 200mM, on incube 5 min à 65°C puis on laisse refroidir le mélange réactionnel jusqu'à température ambiante.

### g) Ligation

On ligue le vecteur de clonage et l'ADNc simple brin dans un volume de 100µl avec 32,5 unités de l'enzyme ADN ligase du phage T4 (Pharmacia ref: 270 87002) pendant une nuit à 15°C.

### h) Synthèse du deuxième brin de l'ADNc

On élimine les protéines par extraction au phénol suivie d'une extraction au chloroforme, puis on ajoute 1/10^{ème} de volume d'une solution d'acétate d'ammonium 10 mM, puis 2,5 volumes d'éthanol. On centrifuge, le culot est dissous dans le tampon de composition Tris acétate pH 7,9, 33 mM, acétate de potassium 62,5 mM, acétate de magnésium 1 mM et dithiothreitol (DTT) 1 mM. Le deuxième brin d'ADN complémentaire est synthétisé dans un volume de 30µl avec 30 unités de l'enzyme ADN polymérase du phage T4 (Pharmacia : réf. 27-0718) et un mélange des quatre déoxynucléotides triphosphates dATP, dCTP, dGTP et dTTP, ainsi que deux unités de la protéine du gène 32 du phage T4 (Pharmacia: réf. 27-0213), pendant 1 h à 37°C. On extrait au phénol et on retire les traces de phénol par une colonne de polyacrylamide P10 (Biogel P10-200-400 mesh- Réf 15011050 - Biorad).

### i) Transformation par électroporation

On transforme des cellules E. coli MC1061 (Clontech) avec l'ADN recombinant obtenu précédemment par électroporation à l'aide de l'appareil Biorad Gene Pulser (Biorad) utilisé à 2,5 kV dans les conditions prescrites par le fabricant, puis on fait pousser les bactéries pendant 6 h 30 dans du milieu dit milieu LB (Sambrook, op cité) de composition : bactotryptone 10 g/l ; extrait de levure 5 g/l ; NaCl 10 g/l, additionné de 100 µg/ml d'ampicilline.

On détermine le nombre de clones indépendants en étalant une dilution au 1/1000^{ème} de la transformation avant l'amplification sur une boîte de milieu LB additionné de 1,5 % d'agar (p/v) et de 100 µg/ml d'ampicilline, appelé par la suite milieu LB gélosé. Le nombre de clones indépendants est de 500 000.

### SECTION 3 : Criblage de la banque d'ADN complémentaire soustraite et sélection du clone NC28

### 1) Réalisation des répliques des colonies bactériennes de la banque d'ADNc sur filtre de nylon :

On distribue environ 40 000 bactéries recombinantes de la banque d'ADNc sur des boîtes de Pétri de (245 x 245) mm contenant du milieu LB gélosé (environ 2000 colonies/boîte).

A partir de chacune de ces boîtes, on réalise un transfert des colonies sur une membrane de nylon (Hybond N-Amersham) par dépôt de la membrane sur la surface de la boîte et mise de repères par perçage de la membrane à l'aide d'une aiguille. La membrane est ensuite retirée et déposée sur la surface d'une nouvelle boîte de Pétri contenant du milieu LB gélosé. On laisse pendant quelques heures à 37°C pour obtenir la repousse des colonies. A partir de cette première membrane on réalise deux répliques sur de nouvelles membranes (préalablement déposées sur du milieu LB gélosé pour les humidifier) par contacts successifs avec la première membrane. Les répliques sur membrane obtenues sont finalement déposées sur des boîtes de milieu LB gélosé et mises à incuber pendant une nuit à 30°C.

Les répliques sur membrane sont déposées avec les colonies vers le haut, sur une feuille de Whatman 3MM saturée avec une solution de composition : NaOH 0,5M; NaCl 1,5M, pendant 5 min, ce qui permet de lyser les bactéries et de fixer l'ADN. Les répliques sur membrane sont ensuite posées sur une deuxième feuille de Whatman 3MM ,saturée cette fois avec une solution neutralisante de composition : NaCl 1,5M ; Tris-HCl pH8 0,5M, pendant 5 min. Les répliques sur membrane sont ensuite plongées dans une solution de 2 x SSC (composition de la solution SSC: NaCl 0,15M ; citrate de sodium 0,015M) et les débris bactériens sont partiellement éliminés en frottant doucement à l'aide d'ouate de nettoyage.

On traite ensuite les répliques sur membrane avec de la protéinase K (Bochringer Mannheim GmbH) à 100 µg/ml dans une solution de composition : Tris-HCl 10mM pH8; EDTA 10mM; NaCl 50mM ; SDS 0,1 % à raison de 20 ml par membrane. On incube pendant30 min à 37°C avec agitation. Les répliques sur membrane sont de nouveau plongées dans une solution de 2 x SSC pour éliminer définitivement toute trace de débris bactériens. Elles sont finalement mises à sécher sur du papier filtre pendant quelques minutes puis pendant 30 min sous vide à + 80°C. On obtient ainsi, pour chaque boîte, deux répliques sur membrane, appelées par la suite réplique 1 et réplique 2.

### 2) Préparation de l'ARN utilisé pour la fabrication des sondes ADNc :

### a) Culture et stimulation de la lignée monocytaire U937

La lignée monocytaire U937 ATCC 1593 est cultivée dans du milieu RPMI complété avec 10 % de sérum de veau foetal (Gibco BRL-réf. 013-06290H), additionné de 10 unités de pénicilline et de 10µg de streptomycine par ml ainsi que de 2 mM final de L-glutamine. Pour l'activation de ces cellules, on les met dans le milieu RPMI additionné de pénicilline, streptomycine et de L-glutamine et de 20 ng/ml de phorbol myristate-2 acétate-3 (PMA) (Sigma-réf. P8139) pendant 24 h. Les cellules ainsi activées sont grattées et centrifugées. Le culot cellulaire obtenu est appelé culot cellulaire U937P.

La moitié des cellules est induite de surcroît avec du cycloheximide (10µg/ml) pendant les deux dernières heures de culture. Cet inhibiteur de la synthèse protéique rend plus stables les ARN messagers instables (dont les cytokines) (cf. T. Lindsten et al., 1989, Science, 244, 339-343). Les cellules ainsi activées sont grattées et centrifugées. Le culot cellulaire obtenu est appelé culot cellulaire U937PC.

### b) Préparation de l'ARN poly A⁺

A partir des culots cellulaires U937P et U937PC, l'ARN est extrait et la fraction polyA⁺ est purifiée comme décrit dans la section 1-2) a) et b). On obtient ainsi deux fractions ARN-polyA⁺, appelées respectivement fraction polyA⁺1 et fraction polyA⁺2.

### 3) Préparation des sondes ADNc radiomarquées :

Les sondes ADNc radiomarquées, appelées respectivement sonde 1 et sonde 2 sont synthétisées à partir des deux fractions ARN-polyA⁺ ci-dessus, préparées comme décrit ci-après.

1 µg d'ARN polyA⁺ est hybridé avec 200 ng d'oligo dT₁₂₋₁₈ (Pharmacia) dans 2 à 3 µl de tampon de composition Tris HCl, pH 7,5, 50 mM et EDTA 1 mM par incubation pendant 2 min à 65°C et refroidissement jusqu'à température ambiante. La synthèse de l'ADNc radiomarqué est réalisée dans un volume réactionnel de 10 µl en tampon de composition : Tris-HCl 50mM pH8,3 ; MgCl₂ 5mM dithiotreitol 10mM, contenant 50µM de dATP dGTP et dTTP, 10 µM de dCTP et 150 µCi de dCTP α32P (3000 Ci/ mmol Amersham) et 40 unités de RNasine (inhibiteur de RNAse-Genofit). La réaction s'effectue à 46°C pendant 30 min en présence de 10 à 20 unités de transcriptase inverse (Genofit). Cette synthèse est suivie de l'hydrolyse alcaline de l'ARN par une solution de NaOH 0,3M dans un volume final de 20µl pendant 30 min à 65°C. On neutralise par ajout d'acide acétique 3M. On ajuste le volume à 50 µl avec du milieu TE. On fait une extraction avec un même volume de phénol suivie d'une deuxième extraction avec un même volume d'un mélange chloroforme/ isoamylalcool (dans les proportions respectives 24/1). On élimine le dCTP α32P non incorporé lors de la synthèse du brin ADNc par chromatographie d'exclusion sur une colonne de polyacrylamide P10 (Biogel-200-400 mesh-Biorad).

La quantité d'ADNc est de 60 à 100 ng possédant une activité spécifique de 1 x 10⁹dpm/µg.

### 4) Hybridation des répliques des colonies bactériennes avec les sondes ADNc:

Les répliques sur membrane sont préhybridées pendant 2 h à 42°C dans un tampon de composition : 50 % formamide ; 6 x SSC; 5 x solution de Denhardt ; 0,1 % SDS et 100 µg/ml d'ADN de sperme de saumon soniqué, rajouté après dénaturation pendant 10 min à 100°C. Les répliques sur membrane sont hybridées pendant deux jours : la réplique 1 avec la sonde 1 et la réplique 2 avec la sonde 2, ces sondes étant utilisées à une concentration de 4 ng/ml dans le tampon ci-dessus. La 5 x solution de Denhardt (cf. Sambrook, op. cité) a la composition : : Ficoll (type 400-Pharmacia) 1 g/l, polyvinylpyrrolidone 1 g/l et la sérumalbumine bovine (BSA) 1 g/l.

Les préhybridation et hybridation s'effectuent en tubes dans un four à hybrider (Hybaid) avec respectivement 25 ml et 10 ml de tampon par membrane.

Ensuite les répliques sur membrane sont successivement lavées plusieurs fois pendant 15 min à 20°C dans le tampon de composition 2 x SSC; 0,1 % SDS, puis deux fois pendant 15 min dans une solution 0,1 x SSC, 0,1 % SDS à 55°C, séchées sur papier Whatman 3MM et autoradiographiées sur films Kodak XAR5.

### 5) Hybridation avec un mélange d'oligonucléotides correspondant à la plupart des cytokines connues :

Pour identifier les clones qui contiennent les ADN complémentaires aux ARN messagers des cytokines connues, une autre série de répliques sur membrane, préparées comme décrit ci-dessus, est hybridée avec un mélange - appelé mélange C - de 28 oligonucléotides comportant chacun 20 nucléotides, correspondant aux ADN complémentaires des cytokines suivantes : Interleukine-1 α(Furutani Y. et al, 1985, Nucl. Ac. Res., 13, 5869-5882), Interleukine-1 β(Auron P. et al, 1984, Proc. Natl. Acad. Sci. USA, 81, 7907-7911), Interleukine-2 (Degrave W. et al, 1983, EMBO J., 2, 3249-3253), Interleukine-3 (Yang Y.C. et al, 1986, 47, 3-10), Interleukine-4 (Yokoto T. et al, 1986, Proc. Ntl. Acad. Sci., 83, 5894-5898), Interleukine-5 (Hirano T. et al, 1986, Nature, 324, 73-75), Interleukine- 6 (May L. et al, 1986, Proc. Natl. Acad. Sci. USA, 83, 8957-8961), Interleukine-7 (Namen A. et al, 1988, Nature, 333, 571-573), Interleukine-8 (Matsushima K. et al, 1988, J. Exp. Med., 167, 1883-1893), Interleukine-9 (Yang Y.C. et al, 1989, Blood, 74, 1880-1884), TNFα (Pennica D. et al, 1984, Nature, 312, 724-729), TNFβ (Gray P. et al, 1984, Nature, 312, 721-724), G-CSF (Nagata S. et al, 1986, 319, 415-418), M-CSF (Kawasaki E. et al, 1985, Science, 230, 291-296), GM-CSF (Wong G. et al, 1985, Science, 228, 810-815), LIF (Grough N. et al, 1988, Proc. Natl. Acad. Sci. USA, 85, 2623-2627), Interféron α (Goeddel D. et al, 1981, Nature, 290, 20-26), Interféron β1 (Taniguchi T. et al, 1980, Gene, 10, 11-15), Interféron γ (Gray P. et al, 1982, Nature, 295, 503-508), TGFα(Derynck R. et al, 1984, Cell, 38, 287-297), TGFβ1 (Derynck R. et al, 1985, Nature, 316, 701-705), bFGF (Prats H. et al, 1989, Proc. Natl. Acad. Sci. USA, 86, 1836-1840), Erythropoiétine (Jacobs K. et al, 1985, Nature, 313, 806-810), BCGF (Sharma S. et al, 1987, Science, 235, 1489-1492), MIF (Weiser W. et al, 1989, Proc. Natl. Acad. Sci USA, 86, 7522-7526), MCP-1 (Yoshimura T. et al, FEBS Lett., 244, 487-493), Oncostatine-M (Malik N. et al, 1989, Mol. Cell. Biol., 9, 2847-2853) et EDF (Murata M. et al, 1988, Proc. Natl. Acad. Sci. USA, 85, 2434-2438).

Ces oligonucléotides, fabriqués à l'aide du synthétiseur d'ADN Biosearch 8700, sont couplés avec de la peroxydase de raifort EC 1.11.17 (Boehringer Mannheim-Réf. 814-407) selon le protocole suivant :
- on fait réagir sur la colonne de synthèse les oligonucléotides avec du carbonyl-diimidazole (Aldrich - 11, 553-3) et du diamino-1,6-hexane (Aldrich - H1.169-6) selon la méthode de Wachter et al, 1986, Nucl. Ac. Res., 14, 7985-7994.
- après déprotection des bases et clivage du support par traitement ammoniacal les oligonucléotides sont purifiés sur une résine d'échange d'ions (Quiagen - Diagen-500051) avec changement du contre-ion ammonium en ion lithium.
- les 5'-amino-oligonucléotides sont couplés à la peroxydase de raifort (Boehringer Mannheim-814407) selon la méthode de M. Urdea et al, Nucl. Ac. Res., 1988, 16, 4937-4956.

Le mélange d'oligonucléotides s'hybride avec environ 10 % des clones de la banque.

Les clones donnant un signal autoradiographique plus fort avec la sonde 2 qu'avec la sonde 1, et ne s'hybridant pas avec le mélange C ont été partiellement séquencés comme décrit dans la section 4 ci-après. Un de ces clones, appelé clone A dans la demande de brevet FR 90 14 961 et désigné par clone NC28 dans la présente demande, a été retenu.

### SECTION 4 : Séquençage et analyse de la séquence de l'ADNc du clone NC28 :

### 1) Séquençage de l'ADNc du clone NC28 :

### a) préparation de l'ADN simple brin

Le clone NC28 contient le vecteur pSE1, lequel porte un ADNc entre les sites ApaI et BamHI, appelé par la suite ADNc NC28.

Le vecteur pSE1, qui contient l'origine de replication du phage f1 permet de produire de l'ADN simple brin par culture du clone NC28 en présence du bactériophage M13K07 (Pharmacia- réf: 27-1524) de la manière suivante :

Le clone NC28 est cultivé, dans un tube de 15 ml, sous agitation à 37°C dans 2 ml de milieu 2 x YT de composition : bactotryptone 16 g/l, extrait de levure 10 g/l NaCl 5 g/l (décrit dans Sambrook et al, op cité), complémenté avec 0,001 % de thiamine et 100 µg/ml d'ampicilline jusqu'à une densité optique à 660 nm d'environ 0,60.
- 100 µl de cette culture sont infectés avec du bactériophage M13K07 (Pharmacia- réf: 27-1524) à une multiplicité d'infection de l'ordre de 10 dans un tube de 15 ml. La culture est mise à agiter à 37°C.
- Au bout de 1 h, 2 ml de milieu sont ajoutés. La culture est alors mise à incuber pendant environ 16 h à 37°C sous agitation.
- 1,5 ml de la culture est centrifugé, dans un microtube, à 15 000 g pendant 2 min.
- 1 ml de surnageant est transféré dans un microtube et additionné de 250 µl d'une solution de polyéthylène glycol (de masse moléculaire 6000) 20 % contenant 2,5M de NaCl. Le mélange est incubé pendant 5 min à 4°C pour faciliter la précipitation du phage puis centrifugé pendant 5 min à 15 000 g. Le surnageant est éliminé et le culot phagique est remis en suspension dans 500 µl de tampon de composition Tris-HCl 10mM pH8, EDTA 1mM.
- La suspension est extraite une fois au phénol saturé avec du Tris-HCl 100mM pH8, puis deux fois au chloroforme.
- La préparation est ensuite précipitée par adjonction de 1/10 de volume d'une solution d'acétate de sodium 3M pH4,8 et 2,5 volumes d'éthanol. La précipitation est réalisée à -20°C pendant un minimum de 20 min. L'ADN est centrifugé pendant 10 min à 15 000 g, le culot est lavé avec une solution d'éthanol à 70 % puis remis en suspension dans 30 µl de tampon de composition : Tris-HCl 10mM pH8, EDTA 1mM.

### b) séquençage

Les réactions de séquençage sont réalisées à l'aide du kit de séquençage United States Biochemical (réf: 70770), qui utilise la méthode de Sanger et al , Proc. Ntl. Acad. Sci. USA, 1977, 14, 5463-5467. Les amorces utilisées sont des oligonucléotides de 18 nucléotides, complémentaires soit au vecteur pSE1 dans la région immédiatement en 5' de l'ADNc NC28, soit à la séquence de l'ADNc NC28.

### 2) Analyse de la séquence de l'ADNc NC28 :

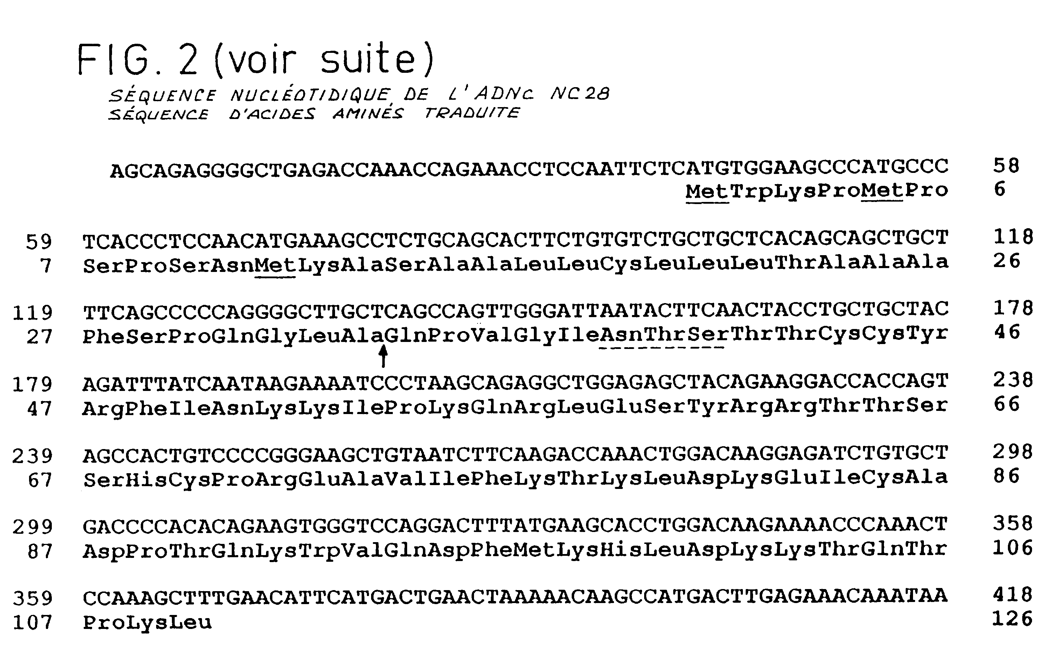

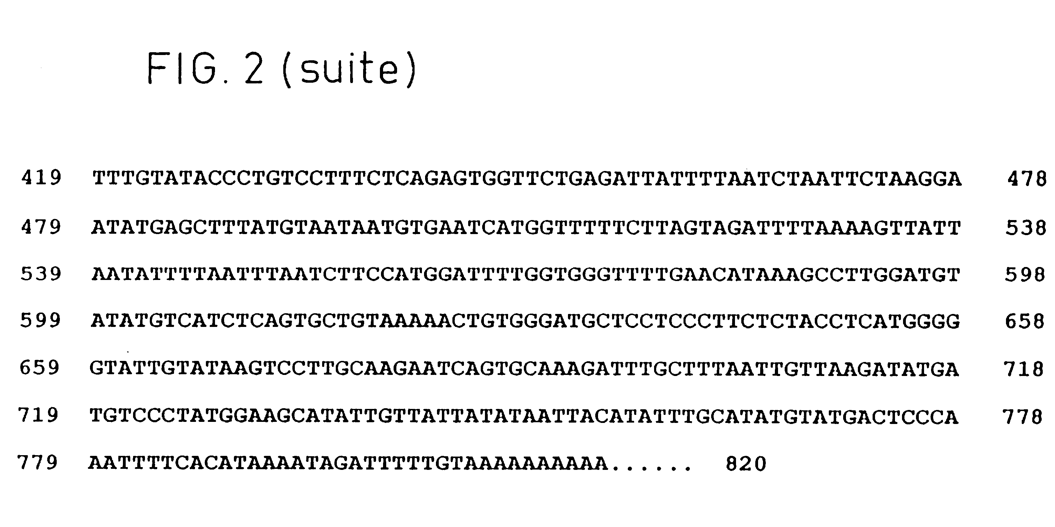

La description ci-après sera mieux comprise à l'aide des figures 2, 3 et 4.

La figure 2 représente la séquence nucléotidique de l'ADNc NC28 et en vis-à-vis la séquence d'acides aminés déduite, les trois Met susceptibles d'initier la traduction étant soulignés, le site de N-glycosylation étant souligné en pointillés et le site de clivage probable du peptide signal étant indiqué par une flèche verticale.

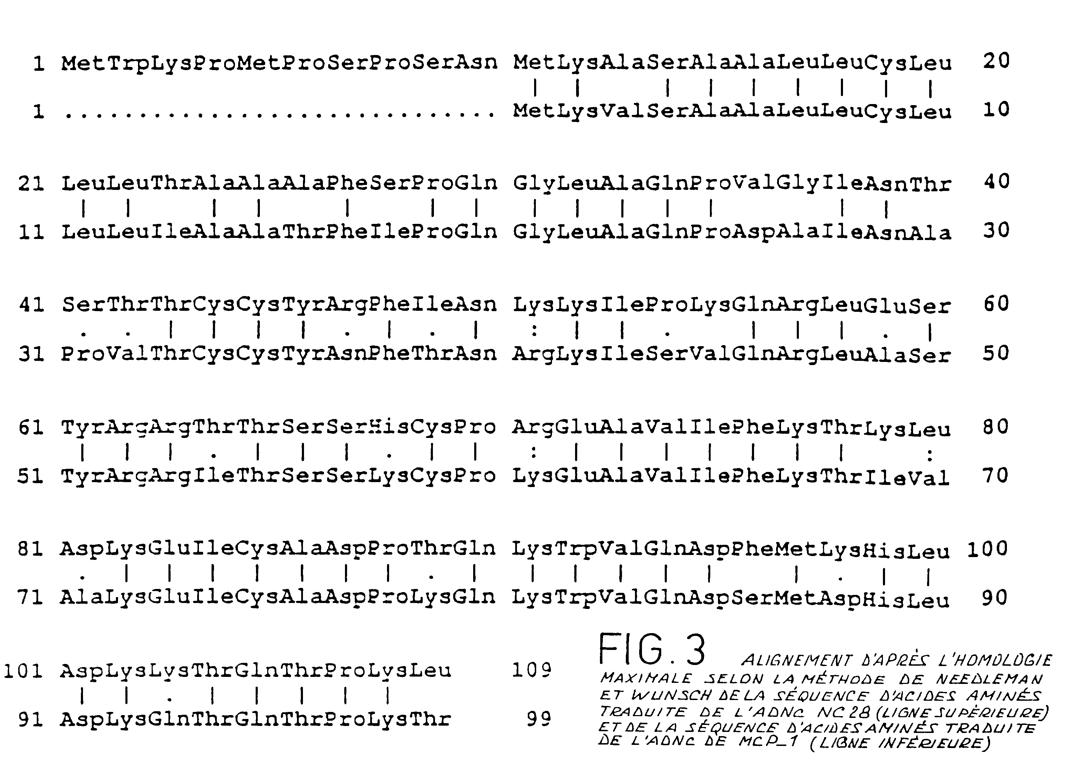

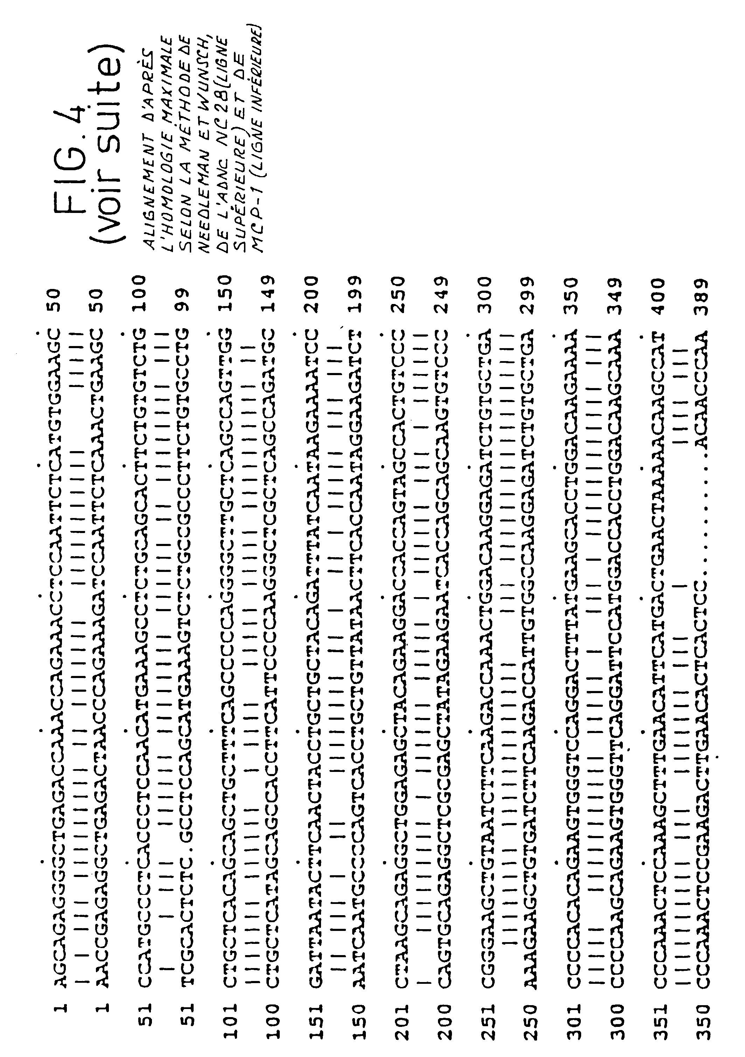

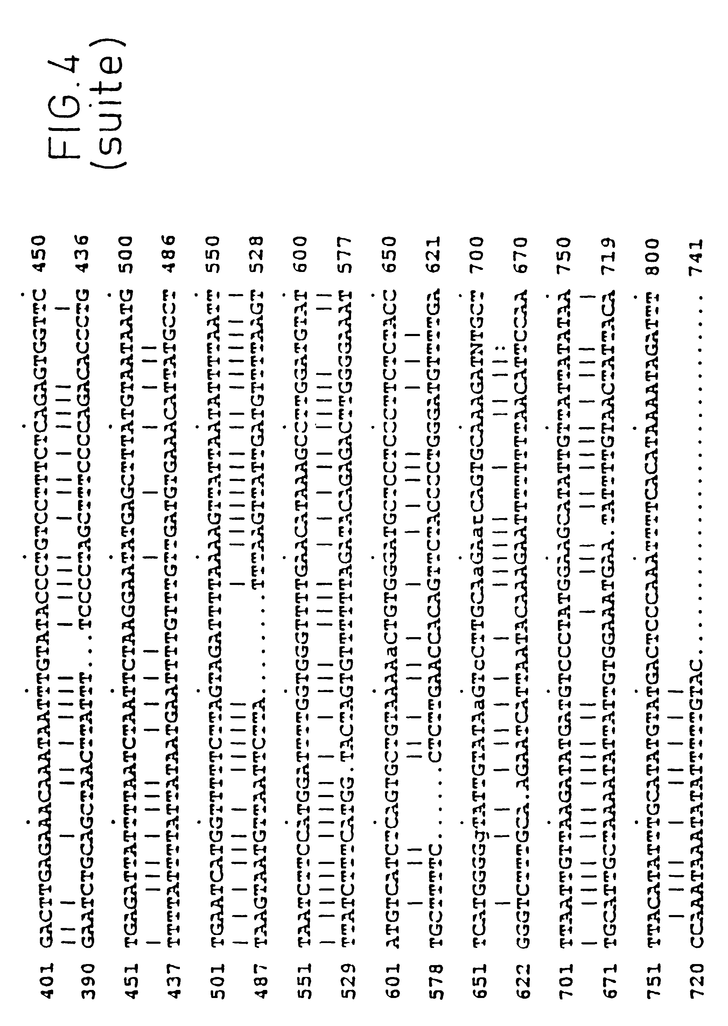

La figure 3 et la figure 4 représentent respectivement l'alignement d'après l'homologie maximale selon la méthode de Needleman et Wunsch, 1970, J. Mol. Biol., 48, 443-453 de la séquence d'acides aminés déduite de l'ADNc NC28 (ligne supérieure) et de la séquence d'acides aminés déduite de l'ADNc de la cytokine MCP-1 (ligne inférieure) et l'alignement selon cette méthode de l'ADNc NC28 (ligne supérieure) et de l'ADNc de la cytokine MCP-1 (ligne inférieure).

### Analyse de la séquence de l'ADNc NC28

(1) L'ADNc NC28 comporte 804 nucléotides et se termine par une séquence poly-A.
(2) Ce nombre de nucléotides est en accord avec la taille de l'ARN messager correspondant (environ 0,8 kb), mesurée par électrophorèse sur gel d'agarose 1 % en présence de formaldéhyde (Sambrook, op cité), suivie d'un transfert sur membrane de nylon (Hybond N+- Amersham) et hybridation selon le protocole décrit ci-après.
   Cette membrane est hybridée avec une sonde radiomarquée avec du ³²P-dCTP (Amersham) fabriquée à partir de l'ADNc NC28 par coupure partielle de ce dernier à l'aide de la DNAse I, suivie d'une polymérisation à l'aide de l'enzyme ADN polymérase I (technique dite de "nick-translation"), comme décrit par Sambrook et al, op cité. L'hybridation se fait à 42°C pendant 16 h en milieu aqueux contenant 50 % de formamide, 1M de NaCl, une solution de 5 x Denhardt et 0,1 % de SDS. Les membranes sont lavées plusieurs fois à température ambiante avec une solution 2 x SSC contenant 0,1 % de SDS, puis lavées deux fois à 50°C pendant 15 min avec une solution 0,1 x SSC contenant 0,1 % de SDS. La solution de 5 x Denhardt a la composition suivante : Ficoll (type 400 - Pharmacia) 1 g/l, polyvinylpyrrolidone 1 g/l et BSA 1 g/l. La solution 1 x SSC contient 0,15M de NaCl et 0,015M de citrate de sodium.
(3) A la position 787-792 la séquence CATAAA, qui ressemble à la séquence consensus AATAAA décrite par M. Birnstiel et al, 1985, Cell, 41, 349, est probablement un signal de polyadénylation. A la position 534-554, une région riche en A et T: TTATTAATATTTTAATTTAAT contient le motif consensus d'instabilité ATTTA décrit par G. Shaw et al, 1986, Cell, 46, 659-667. La plupart des cytokines connues possèdent une telle région riche en A et T, qui contient ce motif consensus d'instabilité.
(4) La séquence d'ADN comporte une phase de lecture ouverte pour la traduction d'une protéine à partir de l'ATG en position 41-43 jusqu'au TGA à la position 368-370 qui correspond à un codon d'arrêt de la traduction. Dans cette phase de lecture il y a trois codons ATG aux positions 41-43, 53-55 et 71-73, susceptibles d'initier la traduction. Parmi ceux-ci, l'environnement nucléotidique de l'ATG aux positions 71-73 est celui qui se rapproche le plus de la séquence consensus décrite par Kozak M., 1978, Cell, 15, 1109-1123, pour l'initiation de la traduction dans les cellules eucaryotes
(5) Un logiciel de recherche de peptide signal, appelé par la suite logiciel PS, a été développé par la Demanderesse d'après la méthode et les informations décrites par Von Heijne, 1986, Nucl. Ac. Res. 14, 483-490. Ce logiciel prévoit dans cette phase de lecture une région hydrophobe ressemblant à un peptide signal et un site probable de clivage protéique à la position 139-140 (entre Ala et Gln). Le peptide signal prévu est compris entre l'une des trois Met susceptibles d'initier la traduction et ce site de clivage. La protéine mature (protéine traduite clivée de son peptide signal) comprend donc 76 acides aminés (cf. figure 2)
(6) La séquence d'acides aminés de la protéine déduite de l'ADNc NC28 et celle de l'ADNc NC28 ont été comparées respectivement à la séquence de la protéine déduite de l'ADNc de la cytokine MCP-1, et à la séquence de l'ADNc de la cytokine MCP-1, à l'aide d'un logiciel approprié, à savoir le logiciel UWGCG de l'Université du Wisconsin : Devereux et al., 1984, Nucl. Ac. Res., 12, 8711-8721 - Option GAP : alignement optimal des séquences d'après la méthode de Needleman et Wunsch, 1970 J. Mol. Biol., 48, 443-453. Cette comparaison a montré environ 74 % d'identité entre la séquence d'acides aminés de la protéine déduite de l'ADNc NC28 et celle de la cytokine MCP-1 (73 acides aminés identiques sur les 99 acides aminés) et environ 79 % d'identité entre la partie de l'ADNc NC28 codant pour la protéine déduite et l'ADNc de la cytokine MCP-1 (235 nucléotides identiques sur les 297 nucléotides).

Le site de clivage prédit par le logiciel PS, à la position 139-140 (entre Ala et Gln) correspond à celui constaté expérimentalement pour la cytokine MCP-1 par E. Robinson et al, 1989, Proc. Ntl. Acad. Sci. USA, 86, 1850-1854. L'ATG en position 71-73 de l'ADNc NC28 correspond à l'ATG initiateur de la traduction de la cytokine MCP-1

L'homologie entre la séquence d'acides aminés de la protéine traduite de l'ADNc NC28 et celle de la cytokine MCP-1 indique que la protéine traduite de l'ADNc NC28 est une protéine sécrétée, de type cytokine.

### SECTION 5 : Analyse de la sécrétion dans les cellules COS de la protéine codée par l'ADNc NC28

Les cellules COS sont des cellules de reins de singe exprimant l'antigène T du virus SV40 (Gluzman, Y. Cell 23, 1981, 175-182). Ces cellules, qui permettent la réplication des vecteurs contenant l'origine de réplication de l'ADN du virus SV40 (cas du vecteur pSE1), constituent des hôtes de choix pour l'étude de l'expression de gènes dans les cellules animales.

### 1) Transfection des cellules COS et expression transitoire de la protéine codée par l'ADNc NC28:

5 x 10⁵ cellules COS sont ensemencées dans une boîte de Pétri de 6 cm de diamètre (Corning) dans 5 ml de milieu modifié d'Eagle selon Dulbecco ci-après appelé DMEM (le Dulbecco's Modified Eagle's medium de Gibco réf.041-01965), lequel contient 0,6 g/l glutamine, 3,7 g/l NaHCO₃ et est complémenté avec du sérum de veau foetal (Gibco) à raison de 5 %. Après environ 16 h de culture à 37°C dans une atmosphère contenant 5 % de dioxyde de carbone, le milieu de culture est enlevé par aspiration et les cellules sont lavées avec 3 ml de tampon PBS (le Phosphate Buffered Saline de GIBCO). On y ajoute alors le mélange suivant : 1000 µl de (DMEM + 10 % sérum de veau foetal (Gibco)), 110 µl de diéthylamino-éthyl-dextrane de poids moléculaire moyen 500 000, à une concentration de 2 mg/ml (Pharmacia), 1,1 µl de chloroquine 100mM (Sigma) et 6 µg d'ADN plasmidique du clone NC28, préparé selon la technique de lyse alcaline suivie de la purification de l'ADN plasmidique sur un gradient de chlorure de césium (Sambrook et al, op cité). Après 5 h d'incubation à 37°C dans une atmosphère contenant 5 % de dioxyde de carbone, le mélange est retiré des cellules. On y ajoute alors 2 ml de tampon PBS contenant 10 % de diméthyl sulfoxyde (qualité Spectroscopie, Merck). Après 1 min d'incubation à la température ambiante, le mélange est retiré et les cellules sont lavées deux fois avec du PBS et sont incubées dans du milieu DMEM contenant 2 % de sérum de veau foetal . L'incubation est poursuivie pendant 40 h à 37°C sous atmosphère contenant 5 % de dioxyde de carbone.

D'autre part, on a préparé des cellules COS témoins en effectuant les opérations décrites ci-dessus avec l'ADN du plasmide pSE1.

### 2) Marquage des protéines :

L'ensemble des opérations décrites ci-dessous est effectué avec les cellules COS transfectées par l'ADN plasmidique du clone NC28 et les cellules COS témoins.

Le milieu de culture est enlevé par aspiration et les cellules sont lavées deux fois avec 3 ml de tampon PBS. On ajoute 5 ml de milieu MEM (Minimal Eagle's Medium) sans méthionine (Gibco - réf.041-01900H), complété avec 3 g/ml de glucose et 4 mM de glutamine. On incube pendant 2 h à 37°C. On enlève le milieu de culture et on rajoute 2 ml de même milieu additionné de 200 µCi de méthionine ³⁵S (réf. SJ1015 Amersham). On incube pendant 6 h à 37°C. On prélève le milieu de culture, on le centrifuge pendant 5 min pour éliminer les débris cellulaires et les cellules en suspension, et on garde le surnageant. Les cellules adhérentes sont rincées deux fois avec du tampon PBS, grattées avec un grattoir en caoutchouc et centrifugées.

### 3) Analyse des protéines radiomarquées des cellules COS transfectées, par électrophorèse sur gel de polyacrylamide :

On précipite 1 ml du surnageant des cellules COS transfectées et 9 ml d'acétone à -20°C. On centrifuge et on récupère les culots de protéines. On les reprend dans un tampon de composition : Tris 0,125M pH 6,8, SDS 4 %, glycérol 20 %. Une partie aliquote de la suspension obtenue correspondant à une radioactivité de 200 000 cpm est analysée par électrophorèse sur un gel de polyacrylamide 15 % en présence de SDS selon la technique décrite par U.K. Laemmli, Anal. Biochem., 1977, 78, 459. Le gel est séché sous vide. Les protéines radiomarquées sont révélées par autoradiographie.

On constate sur l'autoradiogramme la présence de quatre bandes surnuméraires pour les cellules transfectées par l'ADN plasmidique du clone NC28 par rapport aux cellules témoins : deux bandes de forte intensité correspondant à des masses moléculaires apparentes de 9 ± 2 et 16 ± 2 kDa et deux bandes de faible intensité correspondant à des masses moléculaires apparentes de 11 ± 2 et 17 ± 2 kDa (cette dernière bande est diffuse).La masse moléculaire pour la protéine mature de 76 acides aminés est de 8956 kDa, donc proche de la masse moléculaire apparente correspondant à la première de ces bandes.

Les différentes bandes observées ou certaines d'entre elles peuvent correspondre à des degrés de glycosylation divers de la protéine de l'invention. Celle-ci présente en effet (cf. fig. 2) un résidu asparagine susceptible d'être N-glycosylé (souligné en pointillés sur la figure 2 et correspondant à la séquence consensus décrite par DONNER et al. J. Cell. Biol. 1987, 105, 2665) et plusieurs résidus sérine et thréonine susceptibles d'être O-glycosylés.

### 4) Mise en évidence de la N-glycosylation probable des formes de masse moléculaire apparente 16 ± 2 et 17 ± 2 kDa

Le marquage des protéines est effectué comme en 2) ci-dessus mais en présence de 10 µg/ml de tunicanycine (Sigma-réf. T7765), agent inhibiteur de la N-glycosylation des protéines.

L'analyse des protéines sur gel de polyacrylamide est effectuée comme en 3).

On constate sur l'autoradiogramme la présence de deux bandes surnuméraires pour les cellules transfectées par l'ADN plasmidique du clone NC28, par rapport aux cellules témoins : une bande de forte intensité correspondant à une masse moléculaire apparente de 9 ± 2 kDa et une bande de faible intensité correspondant à une masse moléculaire apparente de 11 ± 2 kDa. Ces résultats montrent que les deux formes de la protéine recombinante observées en 3) correspondant à une masse moléculaire de 16 ± 2 et 17 ± 2 kDa sont N-glycosylées.

### SECTION 6 : Purification de la protéine recombinante sécrétée par les cellules COS

### 1) Production de la protéine recombinante

4 x 10⁷ cellules COS sont ensemencées dans un flacon de culture cylindrique, dénommé habituellement "roller", de surface 850 cm² dans 150 ml de milieu modifié d'Eagle selon Dubelcco ci-après appelé DMEM (le Dulbecco's Modified Eagle's medium de Gibco réf: 041-01965), lequel contient 0,6 g/l glutamine, 3,7 g/l NaHCO₃ et est complémenté avec du sérum de veau foetal (Gibco) à raison de 5 % puis tamponné avec du dioxyde de carbone. Après environ 16 h de culture à 37°C avec une vitesse de rotation d'environ 0,2 tr/min, le milieu de culture est enlevé par aspiration et les cellules sont lavées avec du tampon PBS (le Phosphate Buffered Saline de Gibco). On y ajoute alors le mélange suivant : 36 ml de (DMEM + 10 % sérum de veau foetal (Gibco), 4 ml de diéthyl-aminoéthyl-dextrane de poids moléculaire moyen 500 000, à une concentration de 2 mg/ml (Pharmacia), 40 µl de chloroquine 100mM (Sigma) et 128 µg d'ADN plasmidique du clone NC28, préparé selon la technique de lyse alcaline suivie de la purification de l'ADN plasmidique sur un gradient de chlorure de césium (Sambrook et al, op cité). Après 5 h d'incubation à 37°C dans une atmosphère contenant 5 % de dioxyde de carbone, le mélange est retiré des cellules. On y ajoute alors 35 ml de tampon PBS contenant 7 % de diméthyl sulfoxyde (qualité Spectroscopie, Merck). Après 1 min et 30 s de rotation à la température ambiante, le mélange est retiré et les cellules sont lavées deux fois avec du PBS et sont incubées pendant 5 jours à 37°C en rotation dans du milieu DMEM sans rouge de phénol. On prélève le surnageant, de volume environ 130 ml.

### 2) Isolement et purification de la protéine recombinante

La protéine recombinante a été isolée et purifiée à partir du surnageant obtenu ci-dessus, avec les étapes successives suivantes :
- Chromatographie d'échange d'ions sur une colonne DEAE-Sepharose (Pharmacia) préalablement équilibrée avec une solution de Tris-HCl 50 mM pH 8,0. Dans ces conditions opératoires, la protéine ne se fixe pas sur le gel.
- Chromatographie d'affinité sur colonne d'héparine sépharose (Pharmacia) préalablement équilibrée avec une solution de Tris-HCl 50 mM pH 8, avec comme éluant un gradient linéaire de NaCl de 0 à 1 M dans une solution de Tris-HCl 50 mM pH 8,0.
- Dialyse des fractions contenant la protéine recombinante, dont le degré de pureté, déterminé par analyse électrophorétique sur gel de polyacrylamide en présence de SDS, et révélation au nitrate d'argent est supérieur à 90 %, soit contre une solution de PBS pour l'échantillon soumis au test de chimiotactisme décrit dans la section 11, soit contre une solution de tampon MOPS 20 mM pH 6,4 contenant 0,1 M NaCl, appelé solution 1, pour l'étape de chromatographie ci-après.
- Chromatographie d'échange cationique de ces fractions sur une colonne Mono S HR 5/5 (Pharmacia), préalablement équilibrée avec la solution 1, avec comme éluant un gradient linéaire de 0,1 à 0,4 M de NaCl dans la solution 1 (pendant 60 min) et une détection à 280 nm.

La protéine recombinante se trouve dans les fractions correspondant à deux pics, appelés fraction 1 et fraction 2, qui donnent respectivement, par analyse électrophorétique en présence de SDS, pour l'un, une bande majoritaire de masse moléculaire apparente de 9 ± 2 kDa et une bande minoritaire de masse moléculaire apparente de 11 ± 2 kDa, pour l'autre, une bande majoritaire de masse moléculaire apparente de 16 ± 2 kDa et une série de bandes faibles de masses moléculaires apparentes comprise entre (16 et 18) ± 2 kDa.

L'analyse de chacune de ces deux fractions par électrophorèse sur gel de polyacrylamide en présence de SDS et révélation au nitrate d'argent, ne fait pas apparaître d'autres bandes que les formes de la protéine NC28 décrites ci-dessus, ce qui montre que dans chacune des fractions la protéine recombinante a un degré de pureté d'au moins 95 %.

### 3) Analyse de la séquence aminoterminale de chacune des fractions 1 et 2

Pour chacune des fractions 1 et 2, la séquence amino-terminale a été analysée à l'aide d'un séquenceur Applied Biosystem modèle 470A, couplé à un analyseur de dérivés phénylthiohydantoïques Applied Biosystem modele 120A. La protéine purifiée (200 pmoles contrôlées par analyse d'acides aminés) a été déposée sur le séquenceur en présence de 20 pmol de β-lactoglobuline, protéine témoin.

Aucune séquence amino-terminale correspondant à la séquence d'acides aminés codée par l'ADNc NC28 n'a été détectée (par contre la séquence amino-terminale de la protéine témoin a été détectée, donc le séquenceur fonctionne).

Il y a donc probablement un blocage amino-terminal de chacune des différentes formes de la protéine recombinante.

### 4) Etude de la glycosylation des différentes formes de la protéine NC28

Les fractions 1 et 2 ont été soumises à des digestions à l'aide des 3 enzymes, N-glycanase, neuraminidase et O-glycanase (Genzyme), suivant le protocole décrit dans la fiche technique livrée avec ces enzymes.

Les produits de ces digestions enzymatiques ont été analysés par électrophorèse en présence de SDS suivant la technique de Laemmli UK, 1977, Anal Biochem., 78, 459.
Les résultats sont les suivants :

La bande majoritaire de masse moléculaire apparente de 9 ± 2 kDa n'est pas modifiée en présence de N-glycanase, ni de neuraminidase, ni de O-glycanase.

La bande minoritaire de masse moléculaire apparente 11 ± 2 kDa n'est pas modifiée par la N-glycanase, mais est modifiée par la neuraminidase en une bande de masse moléculaire apparente de 10 ± 2 kDa, cette bande étant elle-même modifiée par la O-glycanase en une bande se confondant avec la bande majoritaire de masse moléculaire apparente de 9 ± 2 kDa.

La bande majoritaire de masse moléculaire apparente 16 ± 2 kDa n'est pas modifiée par la neuraminidase, ni par la O-glycanase, mais après action de la N-glycanase, elle se confond avec la bande majoritaire de masse moléculaire apparente de 9 ± 2 kDa.

Les bandes de masses moléculaires apparentes comprises entre (16 et 18) ± 2 kDa, disparaissent sous l'action de chacune des trois enzymes.

Ces expériences montrent que la forme majoritaire de masse moléculaire apparente de 9 ± 2 kDa est une forme de la protéine NC28 non glycosylée, la forme minoritaire de masse moléculaire apparente 11 ± 2 kDa est une forme O-glycosylée de la protéine NC28 contenant 1 ou plusieurs acides sialiques, la forme majoritaire de masse moléculaire apparente 16 ± 2 kDa est une forme N-glycosylée de la protéine NC28.

Les formes minoritaires de masses moléculaires apparentes comprises entre 16 et 18 kDa représentent probablement un mélange complexe de formes N- et O-glycosylées.

La protéine NC28 est le premier membre connu de la famille des cytokines SIS humaines, qui est N-glycosylé (A. Minty, 1991, Médecine Sciences, 7, 578). La cytokine MCP-1 existe dans une forme O-glycosylée mais pas dans une forme N-glycosylée (Jiang et al, 1990, J. Biol. Chem., 30, 18318).

### SECTION 7 : Construction d'un vecteur d'expression de l'ADNc NC28 dans la levure : le plasmide pEMR617 et transformation d'une souche de levure à l'aide de ce plasmide

### 1) Construction du plasmide pEMR617

Le plasmide pEMR583 (décrit dans la demande de brevet EP-435776) a été soumis à une digestion complète par les enzymes HindIII et BamHI. Le grand fragment (appelé ci-après fragment A) comprenant l'origine de replication et le locus STB du 2 micron, le gène LEU2d, le gène de résistance à l'ampicilline, l'origine de pBR322, le terminateur du gène PGK, le gène URA3, le promoteur artificiel et le début de la région prépro de la phéromone alpha a été purifié.

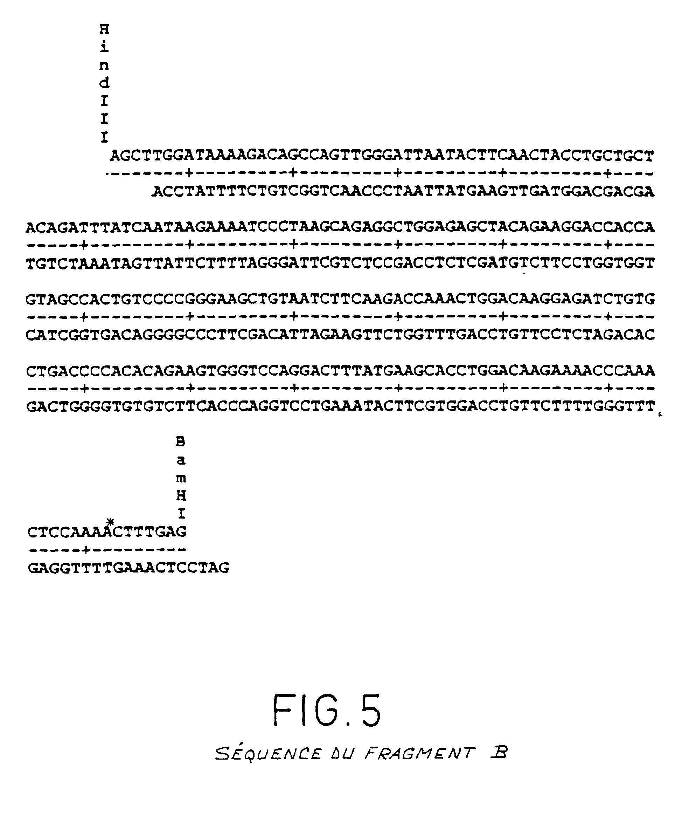

Le fragment HindIII - BamHI (nommé ci-après fragment B) comprenant la fin de la région prépro de la phéromone alpha et l'ADNc codant pour la protéine mature, flanqué du site de restriction BamHI en 3', a été obtenu par amplification par la technique PCR à partir du plasmide pSE₁-NC28 . La séquence de ce fragment est précisée sur la figure 5. Les fragments A et B ont été ligués de façon à obtenir le plasmide pEMR617

### a) Description de la technique de la réaction polymérase en chaîne (Polymerase chain reaction : PCR)

La technique de la réaction de polymérase en chaîne (PCR) est une méthode bien connue de l'homme de l'art qui permet de copier simultanément les deux brins d'une séquence d'ADN préalablement dénaturé en utilisant deux oligonucléotides comme amorces (cf. notamment l'ouvrage de H.A. Erlich : "PCR Technology : Principles and Applications for DNA amplification" publié en 1989 par les éditions Macmillan Publishers Ltd, Royaume-Uni et celui de M.A. Innis et al. "PCR Protocols" publié en 1990 par Academic Press Inc. San Diego, Californie 92101, USA). Le principe de cette technique est résumé ci-après.

La technique du PCR est basée sur la répétition de trois étapes, permettant d'obtenir, après entre 10 et 30 cycles, des centaines de milliers de copies de la matrice originale, à l'aide d'une ADN polymérase de Thermus aquaticus, habituellement appelée polymérase Taq. Les trois étapes sont les suivantes :

### - Dénaturation de la matrice :

L'ADN double brin est dénaturé en ADN simple brin par incubation à haute température (de 92°C à 96°C) pendant approximativement 2 min.

### - Hybridation des amorces :

Ces amorces sont une paire d'oligonucléotides synthétiques qui s'hybrident avec les extrémités de la région à amplifier. Les deux amorces s'hybrident avec les brins opposés. Les amorces sont ajoutées en excès, de façon que la formation du complexe amorce-matrice soit favorisée.

### - Extension des amorces :

L'étape au cours de laquelle la polymérase Taq assure l'extension du complexe amorce-matrice de 5' vers 3' est effectuée à 72°C.

Dans la technique du PCR, le produit d'intérêt apparaît au troisième cycle et il est alors amplifié de manière significative. Au cours du déroulement des cycles, le produit d'amplification devient rapidement la matrice avec laquelle les amorces s'hybrident.

### b) Description des amorces utilisées

Deux oligonucléotides synthétiques ont été préparés.

Le premier oligonucléotide, appelé amorce 1, dont la séquence est la suivante : 
ATCGA AGC TTG GAT AAA AGA     CAG CCA GTT GGG ATT AAT AC
Ser Leu Asp Lys Arg     Gln Pro Val Gly Ile Asn
Région 1     Région 2
 possède deux régions distinctes : la région 1, qui contient la fin de la région prépro de la phéromone a modifiée par rapport à la séquence naturelle décrite par Kurjan et al, Cell, 1982, 30, 933-943 par une mutation silencieuse qui permet d'introduire un site HindIII juste avant la partie codante de la région 1 (cinquième nucléotide de la région 1), et la région 2, qui est une région destinée à s'hybrider avec la région codante correspondant au début de la protéine mature de 76 acides aminés (cf. section 4), du brin non codant de la partie du plasmide pSE1-NC28 qui porte l'ADNc NC28.

Le deuxième oligonucléotide, appelé amorce 2, dont la séquence est la suivante : 
CAGTGGATCC TCAAAGTTTTGGAGTTTGGG
Région 1 Région 2
 est également constitué de deux régions distinctes : la région 1 qui porte sur le cinquième nucléotide un site BamHI, et la région 2, qui porte une séquence nucléotidique correspondant aux derniers codons de la partie codante de l'ADNc-NC28 et au codon stop, avec une mutation destinée à supprimer le site HindIII (mutation silencieuse du codon AAG en AAA). Cette région est destinée à s'hybrider avec le brin codant de la partie du plasmide pSE1-NC28 qui porte l'ADNc NC28.

### c) Obtention du fragment amplifié HindIII - BamHI représentant la fin de la région prépro de la phéromone α et l'ADNc codant pour la protéine mature NC28.

On utilise comme matrice le plasmide pSE1 - NC28 qui porte l'ADNc codant pour la protéine NC28.

Dans un tube on ajoute 100 ng du plamide pSE1 - NC28, 100 ng de l'amorce 1, 100 ng de l'amorce 2, 5 µl de mélange réactionnel concentré 10 fois (quantité finale : 67mM Tris-HCl pH 8,8, 16,6 mM (NH₄)₂SO₄, 1 mM β mercaptoéthanol, 6,7 mM EDTA, 0,15 % Triton x 100, 2 mM MgCl₂, 0,2 mM de dNTP, 200 ng de gélatine) le volume du mélange est ensuite porté à 50 ml en ajoutant de l'eau.

On ajoute 0,5 µl, soit 2,5 unités de Polymérase Taq (Boehringer Manheim réf 1146 - 173). On couvre alors le mélange avec de la paraffine afin d'éviter l'évaporation de la solution aqueuse.

L'amplification se fait au cours de 15 cycles réactionnels, dont les étapes d'un cycle sont les suivantes :
- 1 min à 94°C → dénaturation
- 1 min à 55°C → hybridation
- 1 min à 72°C → polymérisation

Après les 15 cycles, la réaction enzymatique est arrétée par l'addition de 20 mM EDTA.

Le fragment d'ADN ainsi amplifié, qui présente la taille attendue d'environ 250 pb, est ensuite isolé et purifié sur gel d'agarose 1 %, soumis pour dialyse à une chromatographie sur colonne de gel de polyacrylamide P10 (Pharmacia), puis hydrolysé totalement et simultanément par les enzymes HindIII et BamHI selon les techniques habituelles bien connues de l'homme de l'art (Sambrook et al, op cité) afin de former les extrémités cohésives HindIII et BamHI. Après hydrolyse le fragment est purifié sur colonne P10.

La séquence du fragment B obtenu est représentée sur la figure 5. Elle comprend dans sa partie codant pour la protéine NC28 une mutation silencieuse par rapport à l'ADNc NC28, indiquée par un astérisque sur la figure 5.

Les fragments A et B ont été ligués de manière à obtenir le plasmide pEMR617.

### 2) Transformation de la souche de levure EMY761 par le plasmide pEMR617 et expression de la protéine NC28 par la souche transformée

La souche EMY761 (Mat alpha, leu2, ura3, his3) décrite dans le brevet EP-0408461 et pouvant être obtenue par curage plasmidique de la souche déposée à la CNCM le 27 Décembre 1989 sous le n° I-1021, contient des mutations (leu2 et ura3), susceptibles d'être complémentées par le marqueur de sélection défectif LEU2d et le marqueur de sélection URA3, présents dans le plasmide pEMR617. Elle a été transformée par le plasmide pEMR617 avec sélection pour la prototrophie de leucine selon une variante de la technique de transformation décrite par Beggs et al. (Beggs et al, 1978, Nature 275, 104-109) qui consiste à soumettre les levures à un traitement de protoplastisation en présence d'un stabilisant osmotique, le sorbitol en concentration 1 M.

Le protocole précis de transformation est indiqué ci-après :
a) 200 ml du milieu YPG liquide (cf. tableau 1 ci-après) sont inoculés avec environ 5 x 10⁶cellules d'une culture en phase stationnaire et la culture ainsi inoculée est placée pendant une nuit sous agitation à 30°C.
b) Lorsque la culture atteint environ 10⁷ cellules par ml, les cellules sont centrifugées à 4 000 tr/min pendant 5 min et le culot est lavé avec une solution de sorbitol 1 M.
c) Les cellules sont mises en suspension dans 5 ml de solution de sorbitol 1 M contenant 25 mM EDTA et 50 mM de dithiothréitol et incubées pendant 10 min à 30°C.
d) Les cellules sont lavées une fois avec 10 ml de solution de sorbitol 1 M et mises en suspension dans 20 ml de solution de sorbitol. De la zymolase-100T (préparation, commercialisée par Seykagaku Kogyo Co. Ltd., obtenue par purification partielle sur une colonne d'affinité du surnageant de culture d'Arthobacter luteus et contenant de la β-1,3-glucanase-laminaripentahydrolase) est ajoutée à une concentration finale de 20 µg/ml et on incube la suspension à température ambiante pendant environ 15 min.
e) Les cellules sont remises en suspension dans 20 ml d'un milieu contenant du sorbitol appelé milieu YPG sorbitol (cf. tableau I ci-après) et incubées pendant 20 min à 30°C sous agitation douce.
f) On centrifuge pendant 3 min à 2 500 tr/min.
g) On remet en suspension les cellules dans 9 ml de tampon de transformation de composition : sorbitol 1 M, Tris-HCl pH 7,5 10 mM et CaCl₂ 10 mM).
h) On ajoute 0,1 ml de cellules et 5 µl de solution d'ADN (environ 5 mg) et on laisse la suspension obtenue pendant 10 à 15 min à température ambiante.
i) On ajoute 1 ml de la solution : polyéthylène glycol PEG 4000 20 %, Tris-HCl 10 mM pH 7,5 et CaCl₂ 10 mM.
j) On verse 0,1 ml de la suspension obtenue en i) dans un tube contenant du milieu solide de régénération sans leucine (cf. tableau I ci-après) préalablement fondu et maintenu liquide à environ 45°C. On verse la suspension sur une boîte de Pétri contenant une couche solidifiée de 15 ml de milieu de régénération solide sans leucine.

Les transformés commencent à apparaître au bout de trois jours. On a ainsi obtenu un transformé, appelé souche EMY761 pEMR617.

### Tableau 1

### Composition et préparation des principaux milieux utilisés dans le protocole de transformation de la souche de levure EMY761

- milieu YPG liquide
   10 g d'extrait de levure (Bacto-Yeast extract de Difco)
   20 g de peptone (Bacto-peptone de Difco)
   20 g de glucose
   mélanger les ingrédients dans de l'eau distillée. Compléter le volume final à 1 l avec de l'eau distillée - Autoclaver pendant 15 min à 120°C.
- milieu YPG sorbitol
   utiliser la formule du milieu YPG liquide auquel on ajoute, après autoclavage, du sorbitol à une concentration de 1 M.
- milieu solide de régénération sans leucine
   6,7 g de base azotée de levure sans acides aminés (Yeast nitrogen base without Amino Acids de Difco)
   20 mg d'adénine
   20 mg d'uracile
   20 mg de L-tryptophane
   20 mg de L-histidine
   20 mg de L-arginine
   20 mg de L-méthionine
   30 mg de L-tyrosine
   30 mg de L-isoleucine
   30 mg de L-lysine
   50 mg de L-phénylalanine
   100 mg de L-acide glutamique
   150 mg de L-valine
   20 g de glucose
   30 g d'agar
   182 g de sorbitol
   mélanger tous les ingrédients dans l'eau distillée. Compléter le volume final à 1 l, avec de l'eau distillée. Autoclaver pendant 15 min à 120°C. Après autoclavage, ajouter 200 mg de L-thréonine et 100 mg d'acide L-aspartique.

### SECTION 8 : Expression en erlenmeyer de la protéine NC28 par la souche de levure transformée et mise en évidence de la protéine dans le milieu de culture sur gel de polyacrylamide en présence de SDS

### 1) Culture de la souche EMY761 pEMR617

Une colonie de la souche EMY761 pEMR617 (obtenue dans la section 7) a été mise en culture dans 50 ml de milieu liquide sans uracile.
Ce milieu contient pour 1 litre :
- 6,7 g de base azotée de levure sans acides aminés (Yeast nitrogen base without aminoacids de Difco).
- 5,0 g d'hydrolysat de caséine (casaminoacids de Difco)
- 10 g de glucose

Après une nuit à 30°C sous agitation, la culture a été centrifugée pendant 10 min, le culot a été repris dans 10 ml d'eau stérile et de nouveau centrifugée pendant 10 min. L'expression de la protéine NC28 a été induite en reprenant les cellules dans 50 ml de milieu de composition suivante :
- 6,7 g/l de base azotée de levure sans acides aminés (Difco)
- 5,0 g/l d'hydrolysat de caséine (casaaminoacids de Difco)
- 30,0 g/l de glycérol
- 30,0 g/l de galactose
- 10 ml/l d'éthanol.

La culture a été replacée à 30°C sous agitation pendant 24 h.

### 2) Analyse de la protéine exprimée

### a) gel de polyacrylamide en présence de SDS

### Préparation des échantillons

Une partie des cellules cultivées pendant 1 nuit dans un milieu appelé milieu liquide sans uracile avec glucose dont la composition est précisée dans le tableau 2 ci-après, a été centrifugée : échantillon non induit. Les cellules cultivées pendant 1 nuit dans un milieu, appelé milieu liquide sans uracile avec éthanol, glycérol et galactose (tableau 2 ci-après) ont été centrifugées : échantillon induit. Le surnageant a été recueilli. A 10 ml de surnageant, 5 ml d'acide trichloroacétique à 50 % contenant 2 mg/ml de désoxycholate ont été rajoutés.

Le mélange a été mis à la température de + 4°C pendant 30 min, puis centrifugé pendant 30 min. Le culot a été repris dans environ 1 ml d'acétone froid (+ 4°C) et de nouveau centrifugé pendant 30 min. Le culot, après avoir été séché, est repris dans environ 20 µl d'un tampon dénommé tampon de charge constitué de Tris-HCl 0,125 M pH 6,8 SDS 4 %, bleu de bromophénol 0,002 %, glycérol 20 %, β-mercaptoéthanol 10 % selon le protocole décrit par Laemmli (cité précédemment). Le culot est solubilisé par ébullition pendant 15 min puis neutralisé jusqu'à ce que le bleu de bromophénol vire au bleu.

Les échantillons sont déposés sur un gel de polyacrylamide en présence de SDS et soumis à une électrophorèse

### Résultats :

L'analyse du gel (révélation au bleu de Coomassie) montre pour l'échantillon induit la présence de plusieurs bandes supplémentaires par rapport à l'échantillon non induit, dont les deux principales correspondent à un masse moléculaire apparent de 9 ± 2 (forme majoritaire) et 11 ± 2 kDa. Les autres bandes supplémentaires observées d'une masse moléculaire apparente supérieure à 16 ± 2 kDa, qui sont assez nombreuses et diffuses, correspondent probablement à un degré variable de glycosylation. On observe également une bande de faible intensité correspondant à une masse moléculaire apparente de 8 kDa.

On sait que la N-glycosylation d'une protéine par la levure fait intervenir une N-glycosylation simple ("core glycosylation") dans le reticulum endoplasmique et une N-hyperglycosylation ("outer-chain glycosylation") dans l'appareil de Golgi (R.A. Hitzeman et al, 1990, "Methods in Enzymology, n° 185", Academic Press, p. 421-440). En général la N-glycosylation simple conduit à une glyco-protéine de poids moléculaire apparent homogène (une bande) et la N-hyperglycosylation à une glycoprotéine de poids moléculaire apparent hétérogène (pluralité de bandes diffuses). On sait également que certaines protéines peuvent être O-glycosylées par la levure (J. Zueco et al, 1986, Biochemica et Biophysica Acta, 884, 93-100).

La N-glycosylation peut être mise en évidence de plusieurs manières (P. Oleans et al, 1991, Methods in Enzymology, 194, 682-697). L'une d'elles consiste à observer la diminution de la masse moléculaire apparente de la protéine lorsqu'elle est traitée à l'endoglycosydase H (Endo-b-N-acétylglucosaminisidase : E.C. 32.1.96) qui coupe spécifiquement les chaînes N-liées carbohydratées. La O-glycosylation peut être présumée lorsque la protéine est résistante à l'endoglycosydase H et que sa masse moléculaire apparente diminue après traitement à l'α-mannosidase (α-D-manoside mannohydrolase: E.C. 3.2.1.24), comme décrit par Biemans et al, 1991, DNA and Cell Biology, 10, 191-200.

### b) Immunoempreinte (Western blot) avec traitement éventuel à l'endoglycosidase H

### Préparation des échantillons

Une partie des cellules cultivées pendant une nuit en milieu liquide sans uracile avec glucose (tableau 2) a été centrifugée : échantillon non induit.
Les cellules cultivées pendant une nuit en milieu liquide sans uracile avec éthanol, avec glycérol et galactose,(tableau 2) ont été centrifugées : échantillon induit. Le surnageant a été recueilli. A 10 ml de surnageant, 5 ml d'acide trichloroacétique à 50 % contenant 2 mg/ml de désoxycholate ont été rajoutés.

Le mélange a été mis à la température de + 4°C pendant 30 min, puis centrifugé pendant 30 min. Le culot a été repris dans environ 1 ml d'acétone froid (+ 4°C) et de nouveau centrifugé pendant 30 min. Le culot est repris dans 40 µl d'un tampon de solubilisation (de composition: Tris-HCl pH 6.8 10 mM, β mercaptoéthanol 2 %, SDS 1 %). Le culot est porté à 100°C pendant 15 min.

L'échantillon est ensuite partagé en quatre parties
- à la première partie de 10 µl, on ajoute 10 µl d'un tampon citrate de sodium 50 mM pH 5 et 5 µl d'endoglycosidase H (5mUI-Boehringer réf. 1088726). L'échantillon est placé à 37°C pendant environ 1 nuit. On ajoute ensuite 20 µl de tampon de charge ;
- à la deuxième partie de 10 µl, on ajoute 10 µl de tampon citrate de sodium 50 mM pH 5 et 2,5 µl d'α-mannosidase (Sigma Réf. M7257). L'échantillon est placé à 37°C pendant environ 1 nuit. On ajoute ensuite 20 µl de tampon de charge ;
- à la troisième partie de 10 µl, on ajoute 10 µl de tampon citrate de sodium 50 mM pH 5 et 5 µl d'endoglycosidase H (Boehringer). L'échantillon est placé à 37°C pendant environ 1 nuit. On ajoute ensuite 2,5 µl d'α-mannosidase (Sigma). L'échantillon est placé à 37°C pendant 1 nuit. On ajoute 20 µl de tampon de charge ;
- à la quatrième partie de 10 µl, on ajoute 10 µl de tampon de charge. Les échantillons sont portés à ébullition pendant 10 min. On dépose les échantillons sur gel de polyacrylamide en présence de SDS et on effectue une électrophorèse selon le protocole de Laemmli, Anal. Biochem., 1977, 78, 459.

Les protéines contenues dans le gel sont ensuite transférées sur membrane de nitrocellulose (selon la technique de H. Towbin et al, 1979, Proc. Natl. Acad. Sci. USA, 76, 4350-4354). L'immunodétection, réalisée selon le protocole décrit dans l'immuno-Blot Assay Kit de Bio-Rad (réf. 170-6450) implique les étapes suivantes :
- la saturation de la membrane de nitrocellulose avec un tampon TBS ("Tris Buffered Saline") qui contient 3 g/100 ml de gélatine pendant 30 min ;
- le rinçage de la membrane avec un tampon dénommé T. TBS (Tampon TBS qui contient 0,05 % de Tween 20), deux fois pendant 5 min ;
- la mise en contact de la membrane avec l'immunsérum préparé dans la section 10, pendant 1 h à température ambiante ;
- le rinçage de la membrane avec le tampon T.TBS, deux fois pendant 5 min et une fois pendant 5 min avec le tampon TBS ;
- le complexe antigène-anticorps est révélé par la mise en contact de la membrane avec un tampon de développement contenant du chloro-4 naphtol-1 dans du diéthylène et du peroxyde d'hydrogène ;
- le rinçage de la membrane avec de l'eau.

### Résultats :

L'analyse de l'immunoempreinte montre pour l'échantillon induit non traité à l'endoglycosidase H la présence de plusieurs bandes supplémentaires par rapport à l'échantillon non induit, dont les deux principales correspondent à une masse moléculaire apparente de 9 ± 2 et 11 ± 2 kDa. Ces deux bandes sont reconnues par l'immunsérum. Des bandes diffuses de poids moléculaire apparent supérieur à 16 ± 2 kDa sont également mises en évidence.

Dans l'échantillon induit les bandes diffuses correspondant au poids moléculaire supérieur à 16 ± 2 kDa tendent à disparaître après traitement à l'endoglycosidase H pour laisser place à une bande de masse moléculaire apparente de 16 ± 2 kDa pouvant correspondre au précurseur ayant gardé la séquence pro de la phéromone. Dans ce même échantillon traité, on note que les deux bandes principales de poids moléculaires apparents de 9 ± 2 et 11 ± 2 kDa sont toujours présentes.

Dans l'échantillon induit la bande correspondant à un poids moléculaire apparent de 11 ± 2 kDa tend à disparaître après traitement à l'α-mannosidase tandis que la bande de 9 ± 2 kDa augmente en intensité dans les mêmes conditions. Ceci semble indiquer que la protéine correspondant à une masse moléculaire apparente de 11 ± 2 kDa est O-glycosylée.

### Tableau 2

### Composition et préparation de certains milieux utilisés pour la préparation des échantillons :

- Milieu liquide sans uracile avec glucose :
   - 6,7 g de base azotée de levure sans acides aminés (Yeast nitrogen base without amino acids de Difco)
   - 5,0 g d'hydrolysat de caséine (casaminoacids de Difco)
   - 10,0 g de glucose
   mélanger tous les ingrédients dans de l'eau distillée, et compléter à 1 l final avec de l'eau distillée.
   Autoclaver pendant 10 min à 120°C.
- Milieu liquide sans uracile avec éthanol, glycérol et galactose :
   utiliser la formule du milieu liquide sans uracile décrite ci-dessus mais sans glucose. Après autoclavage ajouter 10 ml d'éthanol 100 %, 30 g de glycérol et 30 g de galactose

### SECTION 9 : Purification de la protéine NC28 produite dans la levure et détermination de sa séquence aminoterminale

### 1) Purification de la forme majoritaire de la protéine NC28 produite par levure

La forme majoritaire de la protéine recombinante de masse moléculaire apparente de 9 ± 2 kDa (cf. section 8-2)-a)) a été isolée et purifiée à partir du milieu de culture obtenu à l'issue de la section 8-1), à l'aide d'un protocole comportant les étapes successives suivantes :
- Chromatographie d'échange d'ions sur une colonne DEAE-Sepharose (Pharmacia préalablement équilibrée avec une solution de Tris-HCl 50 mM pH 8,0. Dans ces conditions opératoires, la protéine ne se fixe pas sur le gel.
- Chromatographie d'affinité sur colonne d'héparine sépharose (Pharmacia) préalablement équilibrée avec une solution de Tris-HCl 50 mM pH 8, avec comme éluant un gradient linéaire de NaCl de 0 à 1 M dans une solution de Tris-HCl 50 mM pH 8,0.
- Dialyse des fractions contenant la protéine recombinante (déterminées par analyse électrophorétique sur gel de polyacrylamide en présence de SDS) contre une solution de tampon PBS.

### 2) Analyse de la protéine purifiée

### a) Electrophorèse sur gel de polyacrylamide en présence de SDS

L'analyse par électrophorèse sur gel de polyacrylamide en présence de SDS et révélation au nitrate d'argent montre la présence d'une bande intense correspondant à une masse moléculaire apparente de 9 ± 2 kDa et de deux bandes de très faible intensité correspondant à des masses moléculaires apparentes de 8 ± 2 et 11 ± 2 kDa. La pureté de la protéine recombinante correspondant à ces trois bandes est supérieure à 90 %.

### b) Détermination de la séquence aminoterminale :

La séquence amino-terminale est déterminée selon le principe de la dégradation d'Edman (Kia-Ki Han et al, 1977, Biochimie, 59, 557). Le premier acide aminé amino-terminal est couplé au phényl-isothiocyanate (PITC), puis clivé. Le dérivé obtenu est converti en un phénylthioidantoîne-acide aminé stable, absorbant à 269 nm. Le produit de chaque cycle est analysé par HPLC.

On détecte trois séquences aminoterminales ci-après dans les proportions respectives 70 %, 20 % et 10 % :
. séquence 1 : Gln Pro Val Gly Ilc Asn Thr
. séquence 2 : Val Gly Ile Asn Thr
. séquence 3 :

Lys Ilc Pro Lys Gln

La séquence 1 est la séquence aminoterminale attendue : celle de la protéine mature de 76 acides aminés (cf. figure 2) décrite dans la section 4, dont la séquence codante est introduite dans le vecteur pEMR617 décrit dans la section 7.

Les séquences 1 et 2 sont les séquences amino-terminales de la protéine mature de 76 acides aminés respectivement clivée dans sa partie aminoterminale de 2 et 18 acides aminés.

### SECTION 10 : Synthèse de peptides et production d'immunsérums

### 1) Synthèse de peptides

Plusieurs peptides ont été synthétisés, soit manuellement par la méthode dite du "sachet de thé" ("tea bags"), décrite par Houghten, 1985, Proc. Natl. Acad. Sci-USA, 82, 5131, soit par une méthode utilisant un synthétiseur Milligen. Dans la première méthode, le support de synthèse est enfermé dans une enveloppe perméable et résistante aux solvants ; ainsi, les étapes communes de synthèse (activations, lavages, etc..) peuvent être réalisées simultanément pour un grand nombre de peptides. La deuxième méthode permet de faire une synthèse complètement automatisée. Dans les deux cas, la chimie de synthèse est celle utilisée couramment pour la synthèse de peptides en phase solide (Merrifield et al, 1963, J. Am. Chem Soc. 85, 2149-2154). Dans cette méthode , le résidu carboxy-terminal du peptide à synthétiser est attaché à un polymère insoluble, ensuite différents acides aminés sont ajoutés. La chaîne polypeptidique augmente de taille dans le sens amino-terminal. Après la synthèse, le peptide est séparé du support par l'acide fluorhydrique et récupéré en solution.

Par la première méthode, on a synthétisé les peptides correspondant aux acides aminés 90-109, 94-109 et 97-109 de la protéine traduite NC28 (cf. figure 2), appelés respectivement peptides C20, peptide C16 et peptide C13. Le peptide correspondant aux acides aminés 48-69 de la protéine traduite de l'ADNc NC28, appelé peptide 48-69, a été synthétisé automatiquement. La totalité des peptides a été purifiée par HPLC, la détermination de la composition en acides aminés et le séquençage du peptide ont été effectués sur les produits purifiés.

L'analyse de la composition en acides aminés est entièrement automatisée. L'analyseur d'acides aminés (modèle 420A, Applied Biosystems) effectue l'hydrolyse, puis la dérivation des acides aminés libérés. La séparation des acides aminés dérivés est réalisée par un système HPLC Brownlee 130A connecté en ligne avec le système 420A.

La séquence amino-terminale est déterminée selon le principe de la dégradation d'Edman. Le premier acide aminé amino-terminal est couplé au phényl-isocyanate (PITC), puis clivé. Le dérivé obtenu est converti en un phénylthioidantoîne-acide aminé stable, absorbant à 254 nM. Le produit de chaque cycle est analysé par HPLC.

### 2) Préparation d'immunsérums

Afin de produire des immunsérums, le peptide 48-69 est couplé à une protéine porteuse l'hémocyanine de *Fisurella* (KLH) par son résidu cystéine carboxy-terminal avec le m-maleimidobenzoyl-N-hydroxysuccinimide ester (MBS) comme agent couplant.

Des lapins (New Zealand, mâles de 2 Kg environ) sont immunisés avec le conjugé peptide 48-69/KLH (quantité correspondant à 800 µg de peptide) (lapin N° 43) ou avec 50 µg de protéine NC28 (purifiée à partir de levure - section 9) (lapin N° 44) toutes les deux semaines selon le protocole décrit par Vaitukaitis, 1981, Methods in Enzymology, 73, 46. Pour la première injection, un volume de solution antigénique est émulsifié par un volume d'adjuvant complet de Freund (Sigma-réf. 4258). Les rappels (3 pour le lapin N° 43 et 6 pour le lapin N° 44) sont administrés dans l'adjuvant incomplet de Freund (Sigma-réf. 5506).

Les immunsérums obtenus sont capables de reconnaître la protéine NC28 produite dans la levure et dans les cellules COS par immunodétection après électrophorèse sur gel de polyacrylamide en présence de SDS.

### SECTION 11 : Mise en évidence pour la protéine NC28 et les peptides carboxyterminaux de cette dernière, d'une activité chimiotactique

### 1) Méthode utilisée

### a) Isolement des neutrophiles

A partir du sang périphérique on enlève la plupart des hématies par sédimentation à 37°C pendant 30 min dans une solution contenant 0,6 % dextran T500 (Pharmacia -réf: 17-0320-01) et 0,09 % NaCl. Par la suite les cellules sont déposées par dessus une couche de Ficoll-Paque (Pharmacia) et centrifugées à 400 g pendant 30 min. Les cellules mononucléaires du sang périphérique (PBMNC) sont présentes à l'interface entre le Ficoll et le surnageant alors que les hématies résiduelles et les polynucléaires (principalement des neutrophiles) se trouvent dans le culot cellulaire. Ce culot est remis en suspension dans une solution de NH₄Cl 0,8 %, 10mM Hepes et incubé à 37 °C pendant 7 min pour faire éclater les hématies. Les cellules résiduelles (principalement des neutrophiles) sont centrifugées et lavées dans du tampon HBSS de Hanks Solution Saline équilibrée(Gibco BRL-Réf. 041-04020 H), appelée ci-après solution HBSS.

### b) Isolement des monocytes

Le principe d'isolement des monocytes a été décrit par A. Boyum, 1983, Scan. J. Immunol., 17, 429-436. Il est résumé ci-après. La méthode consiste à séparer les monocytes du sang en utilisant un milieu de gradient iodé, le Nycodenz (N,N'-Bis (dihydroxypropyl-2,3), [N-(dihydroxy propyl-2,3) acétamido]-5-triodo-isophtalamide-2,4,6). Pour accentuer la différence de densité entre les monocytes et des lymphocytes, l'osmolarité de la solution est augmentée,donc les lymphocytes rejettent de l'eau et deviennent plus denses. On peut utiliser du milieu "NycoPrep 1.068", qui contient du Nycodenz, du chlorure de sodium et de la tricine/NaOH à des concentrations optimales pour la séparation des monocytes (Nycomed Pharma AS, Norvège-réf. 223510).

Le protocole utilisé est le suivant :

A partir du sang périphérique on enlève la plupart des hématies par sédimentation à 37°C pendant 30 min dans une solution contenant 0,6 % de dextran et 0,09 % NaCl. La phase supérieure du plasma contenant les monocytes, les lymphocytes et les neutrophiles est prélevée. Pour séparer les monocytes des autres cellules, des tubes sont préparés de la façon suivante : 6 ml de plasma sont déposés sur une couche de 3 ml de NycoPrep 1.068 (Nycomed Pharma AS, Norvège, réf. 223510) dans un tube de diamètre 13-14 mm. Après centrifugation à 600 g pendant 15 min, on prélève le plasma clarifié jusqu'à 3-4 mm au dessus de l'interphase et on recueille le reste du plasma et toute la solution de NycoPrep jusqu'à environ 1 cm au dessus du culot cellulaire, ce qui permet de ne pas prélever les lymphocytes. La suspension de monocytes recueillie est complétée jusqu'à un volume de 6-7 ml avec une solution de composition : 0,9 % NaCl 0,13 % EDTA 1 % BSA, puis centrifugée pendant 7 min à 600 g.

Les monocytes sont contaminés avec des plaquettes. Pour éliminer ces dernières, on centrifuge puis on enlève le surnageant et on remet en suspension avec la même solution, en répétant ces opérations 3 fois.

Les cellules sont remises en suspension dans du milieu RPMI 1640 (Gibco) contenant de l'albumine sérique bovine (BSA) 0,5 %

### c) Protocole de mise en évidence du chimiotactisme

Le test utilisé est celui décrit par W. Falk et al, 1980, J. Imm. Meth., 33, 239-247. Le protocole précis utilisé est exposé ci-après :

On utilise la chambre de Boyden modifiée pour mesure de chimiotactisme, commercialisée par Neuroprobe (réf. AP48). On met les échantillons à tester dilués dans une solution HBSS pour les tests sur les neutrophiles, et le milieu RPMI contenant 0,5 % BSA pour le test sur les monocytes, dans les puits de la plaque inférieure. On dépose sur celle-ci un filtre de polycarbonate (taille des pores : 5 mm-Nuclepore réf. 155845) avec le côté brillant en bas. On dépose la plaque supérieure sur le filtre. On met les cellules (50 000 pour 50 µl tampon) dans les puits de la plaque supérieure. On incube la chambre à 37°C dans une étuve humidifiée ou dans une boîte contenant du coton mouillé pendant 1 h pour le test sur les neutrophiles, et 3 h pour le test sur les monocytes. On retire le filtre et on enlève les cellules qui sont sur le côté mat (cellules qui n'ont pas migré) en essuyant le filtre et en le raclant avec un grattoir en caoutchouc, ces deux dernières opérations étant répétées 1 fois. Les cellules qui ont migré sont colorées et fixées en utilisant le kit "Diff-quick" (Dade-réf. 130832). Par observation microscopique, on compte le nombre de cellules sur le côté brillant du filtre (cellules qui ont migré)

### d) Préparation des échantillons

### a) Echantillons de protéines recombinantes :

- protéine NC28 issue de cellules COS, purifiée comme décrit dans la section 6.
- protéine NC28 issue de levure, purifiée comme décrit dans la section 9
- cytokine MCP-1, obtenue de la façon suivante : isolement d'un plasmide pSEl porteur de l'ADNc de la cytokine MCP-1 par criblage de la banque préparée dans la section 2 à l'aide d'un oligonucléotide correspondant à une partie de l'ADNc de la cytokine MCP-1 décrite par Yoshimura T. et al, 1989, Febs Lett., 244, 487-493, puis transfection des cellules COS, culture des cellules COS transfectées et purification de la cytokine MCP-1, comme décrit pour la protéine NC28.
- protéine IL-8 (Endogen -réf : CY-09025)

### b) Echantillons de peptides :

- peptide C13 ) préparés comme décrit dans
- peptide C16 ) la section 10
- peptide C20 )
- peptide formyl-Met-Leu-Phe, généralement appelé fMLP (Sigma- réf. F 3506)

### 2) Résultats

### a) chimiotactisme de la protéine NC28 purifiée issue des cellules COS et de la protéine NC28 purifiée issue de levure, sur les monocytes

Certains résultats obtenus sont illustrés sur la figure 6a qui représente le nombre de cellules par champ microscopique en fonction de la concentration exprimée en nM pour la protéine NC28 purifiée issue de levure, la protéine NC28 purifiée issue de cellules COS, les peptides C13, C16, C20 et fMLP et sur la figure 6b qui représente le nombre de cellules par champ microscopique en fonction de la concentration exprimée en ng/ml pour la protéine NC28 purifiée issue de levure et la cytokine MCP-1 purifiée issue des cellules COS.

On constate sur la figure 6a que la protéine NC28 purifiée issue de levure et la protéine NC28 purifiée issue de cellules COS, présentent après 3 h d'incubation une activité de chimiotactisme sur les monocytes nettement supérieure à celle du fMLP, peptide connu comme possédant une activité de chimiotactisme sur les monocytes (Schiffmann E. et al, J. Immunol. 114, 1831). Les peptides carboxyterminaux de la protéine NC28 possèdent également cette activité.

On constate sur la figure 6b que l'activité de chimiotactisme sur les monocytes de la protéine NC28 est du même ordre de grandeur que celle de la protéine MCP-1, connue pour cette activité (Rollins B. J. et al, 1991, Blood, 78, 1112) et que cette activité est constatée à des concentrations de 0,1 à 10 ng/ml

### b) Chimiotactisme de la protéine NC28 purifiée issue de levure sur les neutrophiles

Certains résultats obtenus sont illustrés sur la figure 6c, qui représente le nombre de cellules par champ microcospique en fonction de la concentration exprimée en ng/ml, pour la protéine NC28 purifiée issue de levure, la cytokine IL-8 et le peptide fMLP.

On constate que la protéine NC28 ne présente qu'une faible activité de chimiotactisme sur les neutrophiles, (à des concentrations élevées supérieures à 100 ng/ml) contrairement à la cytokine IL-8 et au peptide fMLP, tous deux connus pour posséder cette activité (Yoshimura T. et al, 1987, Proc. Natl. Acad. Sci-USA, 84, 9233,1 et Schiffman E. et al, 1975, J. Immunol., 114, 1831).

La protéine NC28 est donc un chimioattractant puissant et spécifique des monocytes.

## Revendications

1. Protéine présentant une activité de type cytokine, caractérisée en ce qu'elle comprend la séquence (a1) suivante : 
Met Lys His Leu Asp Lys Lys Thr Gln Thr Pro Lys Leu
1               5                   10
 et immédiatement en amont de la séquence (a1), la séquence (a2) suivante : 
Gln Pro Val Gly Ile Asn Thr Ser Thr Thr Cys Cys Tyr Arg Phe Ile
1               5                   10                  15

Asn Lys Lys Ile Pro Lys Gln Arg Leu Glu Ser Tyr Arg Arg Thr Thr
            20                  25                  30

Ser Ser His Cys Pro Arg Glu Ala Val Ile Phe Lys Thr Lys Leu Asp
        35                  40                  45

Lys Glu Ile Cys Ala Asp Pro Thr Gln Lys Trp Val Gln Asp Phe
    50                  55                  60
 ou une partie de la séquence (a₂).

2. Protéine selon la revendication 1, caractérisée en ce qu'elle comprend immédiatement en amont de la séquence (a1), une partie de la séquence (a2).

3. Protéine selon la revendication 2, caractérisée en ce que la partie de la séquence (a2) immédiatement en amont de la séquence (a1) est choisie parmi les séquences suivantes :

Gln Lys Trp Val Gln Asp Phe et Gln Asp Phe

4. Protéine selon la revendication 2, caractérisée en ce que la partie de la séquence (a2) immédiatement en amont de la séquence (a1) est choisie parmi les séquences (a3) et (a4) suivantes :

(a3)

Val Gly Ile Asn Thr Ser Thr Thr Cys Cys Tyr Arg Phe Ile Asn Lys
1               5                   10                  15

Lys Ile Pro Lys Gln Arg Leu Glu Ser Tyr Arg Arg Thr Thr Ser Ser
            20                  25                  30

His Cys Pro Arg Glu Ala Val Ile Phe Lys Thr Lys Leu Asp Lys Glu
        35                  40                  45

Ile Cys Ala Asp Pro Thr Gln Lys Trp Val Gln Asp Phe
    50                  55                  60

(a4)

Lys Ile Pro Lys Gln Arg Leu Glu Ser Tyr Arg Arg Thr Thr Ser Ser
1               5                   10                  15

His Cys Pro Arg Glu Ala Val Ile Phe Lys Thr Lys Leu Asp Lys Glu
            20                  25                  30

Ile Cys Ala Asp Pro Thr Gln Lys Trp Val Gln Asp Phe
        35                  40                  45

5. Protéine selon la revendication 1, caractérisée en ce qu'elle comporte un blocage aminoterminal.

6. Protéine selon la revendication 1, caractérisée en ce qu'elle a une masse moléculaire apparente de 9 ± 2 kDa.

7. Protéine selon la revendication 1, caractérisée en ce qu'elle a une masse moléculaire apparente de 11 ± 2 kDa.

8. Protéine selon la revendication 1, caractérisée en ce qu'elle a une masse moléculaire apparente de 16 ± 2 kDa.

9. Protéine selon la revendication 7, caractérisée en ce qu'elle est O-glycosylée.

10. Protéine selon la revendication 8, caractérisée en ce qu'elle est N-glycosylée.

11. Protéine selon la revendication 1, caractérisée en ce qu'elle a un degré de pureté supérieur à 90 %.

12. Protéine selon la revendication 11, caractérisée en ce qu'elle a un degré de pureté supérieur à 95 %.

13. ADN recombinant caractérisé en ce qu'il code pour une protéine selon la revendication 1 ou pour un précurseur de cette protéine, qui comprend une séquence signal.

14. ADN recombinant selon la revendication 13, caractérisé en ce que la séquence signal est une séquence choisie parmi les séquences (b1), (b2) et (b3) suivantes :

(b1)    Met Lys Ala Ser Ala Ala Leu Leu Cys Leu Leu Leu Thr Ala Ala Ala
        1               5                   10                  15
        Phe Ser Pro Gln Gly Leu Ala
                    20

(b2) Met Pro Ser Pro Ser Asn Met Lys Ala Ser Ala Ala Leu Leu Cys Leu
1 5 10 15
Leu Leu Thr Ala Ala Ala Phe Ser Pro Gln Gly Leu Ala
20 25

(b3) Met Trp Lys Pro Met Pro Ser Pro Ser Asn Met Lys Ala Ser Ala Ala
1 5 10 15
Leu Leu Cys Leu Leu Leu Thr Ala Ala Ala Phe Ser Pro Gln Gly Leu
20 25 30
Ala

15. ADN recombinant selon la revendication 14, caractérisé en ce que la séquence nucléotidique codant pour la protéine mature est la séquence (Na2) suivante :

CAGCCAGTTG GGATTAATAC TTCAACTACC TGCTGCTACA
GATTTATCAA TAAGAAAATC CCTAAGCAGA GGCTGGAGAG
CTACAGAAGG ACCACCAGTA GCCACTGTCC CCGGGAAGCT
GTAATCTTCA AGACCAAACT GGACAAGGAG ATCTGTGCTG
ACCCCACACA GAAGTGGGTC CAGGACTTTA TGAAGCACCT
GGACAAGAAA ACCCAAACTC CAAAGCTT

16. ADN recombinant selon la revendication 15, caractérisé en ce que les séquences nucléotidiques codant pour les séquences d'acides aminés (b1), (b2) et (b3) sont choisies parmi les séquences (Nb1), (Nb2) et (Nb3) suivantes :

(Nb1) ATGAAAGCCT CTGCAGCACT TCTGTGTCTG CTGCTCACAG
CAGCTGCTTT CAGCCCCCAG GGGCTTGCT

(Nb2) ATGCCCTCAC CCTCCAACAT GAAAGCCTCT GCAGCACTTC
TGTGTCTGCT GCTCACAGCA GCTGCTTTCA GCCCCCAGGG
GCTTGCT

(Nb3) ATGTGGAAGC CCATGCCCTC ACCCTCCAAC ATGAAAGCCT
CTGCAGCACT TCTGTGTCTG CTGCTCACAG CAGCTGCTTT
CAGCCCCCAG GGGCTTGCT

17. Vecteur d'expression, caractérisé en ce qu'il contient, avec les moyens nécessaires à son expression, un ADN recombinant selon l'une des revendications 13 à 16.

18. Cellules animales, caractérisées en ce qu'elles contiennent un vecteur d'expression selon la revendication 17.

19. Cellules animales, caractérisées en ce qu'elles contiennent, avec les moyens nécessaires à son expression, un ADN recombinant selon l'une des revendications 14 à 16.

20. Cellules animales selon la revendication 18 ou 19, caractérisées en ce qu'elles sont des cellules CHO.

21. Cellules animales selon la revendication 18 ou 19, caractérisées en ce qu'elles sont des cellules COS.

22. Levure caractérisée en ce qu'elle contient, avec les moyens nécessaires à son expression, un ADN recombinant selon l'une des revendications 13 à 16.

23. Procédé de préparation d'une protéine selon l'une des revendications 1 à 12, caractérisé en ce qu'il comprend une étape de culture de cellules animales selon la revendication 20 ou 21, suivie de l'isolement et de la purification de la protéine recombinante.

24. Procédé de préparation d'une protéine selon l'une des revendications 1 à 12, caractérisée en ce qu'il comprend une étape de culture de levure selon la revendication 22 suivie de l'isolement et de la purification de la protéine recombinante.

25. Médicament caractérisé en ce qu'il contient une protéine selon l'une des revendications 1 à 12.

## Patentansprüche

1. Protein mit Cytokinaktivität, dadurch gekennzeichnet, dass es die folgende Sequenz (a1) aufweist: 
Met Lys His Leu Asp Lys Lys Thr Gln Thr Pro Lys Leu
1               5                   10
 und der Sequenz (a1) unmittelbar vorgeordnet die folgende Sequenz (a2): 
Gln Pro Val Gly Ile Asn Thr Ser Thr Thr Cys Cys Tyr Arg Phe Ile
1               5                   10                  15

Asn Lys Lys Ile Pro Lys Gln Arg Leu Glu Ser Tyr Arg Arg Thr Thr
            20                  25                  30

Ser Ser His Cys Pro Arg Glu Ala Val Ile Phe Lys Thr Lys Leu Asp
        35                  40                  45

Lys Glu Ile Cys Ala Asp Pro Thr Gln Lys Trp Val Gln Asp Phe
    50                  55                  60
 oder einen Teil der Sequenz (a2).

2. Protein nach Anspruch 1, dadurch gekennzeichnet, dass es der Sequenz (a1) unmittelbar vorgeordnet einen Teil der Sequenz (a2) aufweist.

3. Protein nach Anspruch 2, dadurch gekennzeichnet, dass der der Sequenz (a1) unmittelbar vorgeordnete Teil der Sequenz (a2) ausgewählt ist aus den folgenden Sequenzen:

Gln Lys Trp Val Gln Asp Pheund Gln Asp Phe

4. Protein nach Anspruch 2, dadurch gekennzeichnet, dass der der Sequenz (a1) unmittelbar vorgeordnete Teil der Sequenz (a2) ausgewählt ist aus den folgenden Sequenzen (a3) und (a4):

(a3)

Val Gly Ile Asn Thr Ser Thr Thr Cys Cys Tyr Arg Phe Ile Asn Lys
1               5                   10                  15

Lys Ile Pro Lys Gln Arg Leu Glu Ser Tyr Arg Arg Thr Thr Ser Ser
            20                  25                  30

His Cys Pro Arg Glu Ala Val Ile Phe Lys Thr Lys Leu Asp Lys Glu
        35                  40                  45

Ile Cys Ala Asp Pro Thr Gln Lys Trp Val Gln Asp Phe
    50                  55                  60

(a4)

Lys Ile Pro Lys Gln Arg Leu Glu Ser Tyr Arg Arg Thr Thr Ser Ser
1               5                   10                  15

His Cys Pro Arg Glu Ala Val Ile Phe Lys Thr Lys Leu Asp Lys Glu
            20                  25                  30

Ile Cys Ala Asp Pro Thr Gln Lys Trp Val Gln Asp Phe
        35                  40                  45

5. Protein nach Anspruch 1, dadurch gekennzeichnet, dass es eine Blockierung am Aminoende aufweist.

6. Protein nach Anspruch 1, dadurch gekennzeichnet, dass es ein scheinbares Molekulargewicht von 9 ± 2 kDa hat.

7. Protein nach Anspruch 1, dadurch gekennzeichnet, dass es ein scheinbares Molekulargewicht von 11 ± 2 kDa hat.

8. Protein nach Anspruch 1, dadurch gekennzeichnet, dass es ein scheinbares Molekulargewicht von 16 ± 2 kDa hat.

9. Protein nach Anspruch 7, dadurch gekennzeichnet, dass es O-glycosyliert ist.

10. Protein nach Anspruch 8, dadurch gekennzeichnet, dass es N-glycosyliert ist.

11. Protein nach Anspruch 1, dadurch gekennzeichnet, dass es einen Reinheitsgrad von über 90 % aufweist.

12. Protein nach Anspruch 11, dadurch gekennzeichnet, dass es einen Reinheitsgrad von über 95 % aufweist.

13. Rekombinante DNA, dadurch gekennzeichnet, dass sie für ein Protein nach Anspruch 1 oder einen Vorläufer dieses Proteins codiert, der eine Signalsequenz aufweist.

14. Rekombinante DNA nach Anspruch 13, dadurch gekennzeichnet, dass die Signalsequenz eine Sequenz ist, die ausgewählt ist aus den folgenden Sequenzen (bl), (b2) und (b3):

(b1) Met Lys Ala Ser Ala Ala Leu Leu Cys Leu Leu Leu Thr Ala Ala Ala
1 5 10 15
Phe Ser Pro Gln Gly Leu Ala
20

(b2) Met Pro Ser Pro Ser Asn Met Lys Ala Ser Ala Ala Leu Leu Cys Leu
1 5 10 15
Leu Leu Thr Ala Ala Ala Phe Ser Pro Gln Gly Leu Ala
20 25

(b3) Met Trp Lys Pro Met Pro Ser Pro Ser Asn Met Lys Ala Ser Ala Ala
1 5 10 15
Leu Leu Cys Leu Leu Leu Thr Ala Ala Ala Phe Ser Pro Gln Gly Leu
20 25 30
Ala

15. Rekombinante DNA nach Anspruch 14, dadurch gekennzeichnet, dass die für das reife Protein codierende Nucleotidsequenz die folgende Sequenz (Na2) ist:

CAGCCAGTTG GGATTAATAC TTCAACTACC TGCTGCTACA
GATTTATCAA TAAGAAAATC CCTAAGCAGA GGCTGGAGAG
CTACAGAAGG ACCACCAGTA GCCACTGTCC CCGGGAAGCT
GTAATCTTCA AGACCAAACT GGACAAGGAG ATCTGTGCTG
ACCCCACACA GAAGTGGGTC CAGGACTTTA TGAAGCACCT
GGACAAGAAA ACCCAAACTC CAAAGCTT

16. Rekombinante DNA nach Anspruch 15, dadurch gekennzeichnet, dass die für die Aminosäuresequenzen (b1), (b2) und (b3) codierenden Nucleotidsequenzen ausgewählt sind aus den folgenden Sequenzen (Nb1), (Nb2) und (Nb3):

(Nb1)
ATGAAAGCCT CTGCAGCACT TCTGTGTCTG CTGCTCACAG
CAGCTGCTTT CAGCCCCCAG GGGCTTGCT

(Nb2)
ATGCCCTCAC CCTCCAACAT GAAAGCCTCT GCAGCACTTC
TGTGTCTGCT GCTCACAGCA GCTGCTTTCA GCCCCCAGGG
GCTTGCT

(Nb3)
ATGTGGAAGC CCATGCCCTC ACCCTCCAAC ATGAAAGCCT
CTGCAGCACT TCTGTGTCTG CTGCTCACAG CAGCTGCTTT
CAGCCCCCAG GGGCTTGCT

17. Expressionsvektor, dadurch gekennzeichnet, dass er zusammen mit den für ihre Expression notwendigen Mitteln eine rekombinante DNA nach einem der Ansprüche 13 bis 16 enthält.

18. Tierische Zellen, dadurch gekennzeichnet, dass sie einen Expressionsvektor nach Anspruch 17 enthalten.

19. Tierische Zellen, dadurch gekennzeichnet, dass sie zusammen mit den für ihre Expression notwendigen Mitteln eine rekombinante DNA nach einem der Ansprüche 14 bis 16 enthalten.

20. Tierische Zellen nach Anspruch 18 oder 19, dadurch gekennzeichnet, dass es CHO-Zellen sind.

21. Tierische Zellen nach Anspruch 18 oder 19, dadurch gekennzeichnet, dass es COS-Zellen sind.

22. Hefe, dadurch gekennzeichnet, dass sie zusammen mit den für ihre Expression notwendigen Mitteln eine rekombinante DNA nach einem der Ansprüche 13 bis 16 enthält.

23. Verfahren zur Herstellung eines Proteins nach einem der Ansprüche 1 bis 12, dadurch gekennzeichnet, dass es einen Schritt des Kultivierens von tierischen Zellen nach Anspruch 20 oder 21 mit anschließendem Isolieren und Reinigen des rekombinanten Proteins umfasst.

24. Verfahren zur Herstellung eines Proteins nach einem der Ansprüche 1 bis 12, dadurch gekennzeichnet, dass es einen Schritt des Kultivierens von Hefe nach Anspruch 22 mit anschließendem Isolieren und Reinigen des rekombinanten Proteins umfasst.

25. Medikament, dadurch gekennzeichnet, dass es ein Protein nach einem der Ansprüche 1 bis 12 enthält.

## Claims

1. A protein having a cytokine-type activity, which comprises the following sequence (a1) : 
Met Lys His Leu Asp Lys Lys Thr Gln Thr Pro Lys Leu
1               5                   10
 and, immediately upstream of the sequence (a1), the following sequence (a2) : 
Gln Pro Val Gly Ile Asn Thr Ser Thr Thr Cys Cys Tyr Arg Phe Ile
1               5                   10                  15

Asn Lys Lys Ile Pro Lys Gln Arg Leu Glu Ser Tyr Arg Arg Thr Thr
            20                  25                  30

Ser Ser His Cys Pro Arg Glu Ala Val Ile Phe Lys Thr Lys Leu Asp
        35                  40                  45

Lys Glu Ile Cys Ala Asp Pro Thr Gln Lys Trp Val Gln Asp Phe
    50                  55                  60
 or a part of the sequence (a2).

2. The protein according to claim 1, which comprises, immediately upstream of the sequence (a1), a part of the sequence (a2).

3. The protein according to claim 2 wherein the part of the sequence (a2) immediately upstream of the sequence (a1) is selected from the following sequences :

Gln Lys Trp Val Gln Asp Phe and Gln Asp Phe

4. The protein according to claim 2 wherein the part of the sequence (a2) immediately upstream of the sequence (a1) is selected from the following sequences (a3) and (a4) :

(a3)

Val Gly Ile Asn Thr Ser Thr Thr Cys Cys Tyr Arg Phe Ile Asn Lys
1               5                   10                  15

Lys Ile Pro Lys Gln Arg Leu Glu Ser Tyr Arg Arg Thr Thr Ser Ser
            20                  25                  30

His Cys Pro Arg Glu Ala Val Ile Phe Lys Thr Lys Leu Asp Lys Glu
        35                  40                  45

Ile Cys Ala Asp Pro Thr Gln Lys Trp Val Gln Asp Phe
    50                  55                  60

(a4)

Lys Ile Pro Lys Gln Arg Leu Glu Ser Tyr Arg Arg Thr Thr Ser Ser
1               5                   10                  15

His Cys Pro Arg Glu Ala Val Ile Phe Lys Thr Lys Leu Asp Lys Glu
            20                  25                  30

Ile Cys Ala Asp Pro Thr Gln Lys Trp Val Gln Asp Phe
        35                  40                  45

5. The protein according to claim 1 which has amino-terminal blocking.

6. The protein according to claim 1 which has an apparent molecular weight of 9 ± 2 kDa.

7. The protein according to claim 1 which has an apparent molecular weight of 11 t 2 kDa.

8. The protein according to claim 1 which has an apparent molecular weight of 16 ± 2 kDa.

9. The protein according to claim 7 which is 0-glycosylated.

10. The protein according to claim 8 which is N-glycosylated.

11. The protein according to claim 1 which has a degree of purity of more than 90 %.

12. The protein according to claim 11 which has a degree of purity of more than 95 %.

13. A recombinant DNA which codes for a protein according to claim 1 or for a precursor of said protein which comprises a signal sequence.

14. The recombinant DNA according to claim 13 wherein the signal sequence is a sequence selected from the following sequences (b1), (b2) and (b3) :

(b1) Mct Lys Ala Ser Ala Ala Leu Leu Cys Leu Leu Lcu Thr Ala Ala Ala
1 5 10 15
Phe Ser Pro Gln Gly Leu Ala
20

(b2) Met Pro Scr Pro Ser Asn Met Lys Ala Ser Ala Ala Lcu Lcu Cys Lcu
1 5 10 15
Leu Leu Thr Ala Ala Ala Phe Ser Pro Gln Gly Lcu Ala
20 25

(b3) Met Trp Lys Pro Met Pro Ser Pro Ser Asn Met Lys Ala Ser Ala Ala
1 5 10 15
Leu Leu Cys Leu Leu Leu Thr Ala Ala Ala Phe Ser Pro Gln Gly Leu
20 25 30
Ala

15. The recombinant DNA according to claim 14 wherein the nucleotide sequence coding for the mature protein is the following sequence (Na2) :

CAGCCAGTTG GGATTAATAC TTCAACTACC TGCTGCTACA
GATTTATCAA TAAGAAAATC CCTAAGCAGA GGCTGGAGAG
CTACAGAAGG ACCACCAGTA GCCACTGTCC CCGGGAAGCT
GTAATCTTCA AGACCAAACT GGACAAGGAG ATCTGTGCTG
ACCCCACACA GAAGTGGGTC CAGGACTTTA TGAAGCACCT
GGACAAGAAA ACCCAAACTC CAAAGCTT

16. The recombinant DNA according to claim 15 wherein the nucleotide sequences coding for the amino acid sequences (b1), (b2) and (b3) are selected from the following sequences (Nb1), (Nb2) and (Nb3) :

(Nb1) ATGAAAGCCT CTGCAGCACT TCTGTGTCTG CTGCTCACAG
CAGCTGCTTT CAGCCCCCAG GGGCTTGCT

(Nb2) ATGCCCTCAC CCTCCAACAT GAAAGCCTCT GCAGCACTTC
TGTGTCTGCT GCTCACAGCA GCTGCTTTCA GCCCCCAGGG
GCTTGCT

(Nb3) ATGTGGAAGC CCATGCCCTC ACCCTCCAAC ATGAAAGCCT
CTGCAGCACT TCTGTGTCTG CTGCTCACAG CAGCTGCTTT
CAGCCCCCAG GGGCTTGCT

17. An expression vector which contains a recombinant DNA according to one of claims 13 to 16, together with the means necessary for its expression.

18. Animal cells which contain an expression vector according to claim 17.

19. Animal cells which contain a recombinant DNA according to one of claims 14 to 16, together with the means necessary for its expression.

20. Animal cells according to claim 18 or 19 which are CHO cells.

21. Animal cells according to claim 18 or 19 which are COS cells.

22. A yeast which contains a recombinant DNA according to one of claims 13 to 16, together with the means necessary for its expression.

23. A method of preparing a protein according to one of claims 1 to 12, which comprises a step of culturing animal cells according to claim 20 or 21, followed by the isolation and purification of the recombinant protein.

24. A method of preparing a protein according to one of claims 1 to 12, which comprises a step of culturing the yeast according to claim 22, followed by the isolation and purification of the recombinant protein.

25. A medicament which contains a protein according to one of claims 1 to 12.
